# EUROPÄISCHE PATENTSCHRIFT

(11) **EP 0 854 861 B1**
(45) Veröffentlichungstag und Bekanntmachung des Hinweises auf die Patenterteilung: **25.07.2001**
(21) Anmeldenummer: 96933424.2
(22) Anmeldetag: 30.09.1996
(51) Int. Cl.: C07C 259/12, C07C 259/14, A01N 37/52, C07C 327/56

(54) **OXYAMINOOXIMETHER, VERFAHREN UND ZWISCHENPRODUKTE ZU IHRER HERSTELLUNG UND SIE ENTHALTENDE MITTEL ZUR BEKÄMPFUNG VON SCHADPILZEN UND TIERISCHEN SCHÄDLINGEN**
OXYAMINO OXIME ETHERS, METHODS AND INTERMEDIATE PRODUCTS FOR THEIR PRODUCTION, AND AGENTS CONTAINING THEM AND USED FOR CONTROLLING HARMFUL FUNGI AND PESTS
ETHERS D'OXYAMINO-OXIME, PROCEDES ET PRODUITS INTERMEDIAIRES UTILISES POUR LEUR PRODUCTION ET AGENT LES CONTENANT SERVANT A LUTTER CONTRE LES CHAMPIGNONS PARASITES ET LES PARASITES ANIMAUX

(30) Priorität: 10.10.1995 DE 19537750
(43) Veröffentlichungstag der Anmeldung: 29.07.1998
(73) Patentinhaber: BASF AKTIENGESELLSCHAFT, 67056 Ludwigshafen (DE)
(72) Erfinder: BAYER, Herbert, D-68159 Mannheim (DE); SAUTER, Hubert, D-68167 Mannheim (DE); OBERDORF, Klaus, D-69117 Heidelberg (DE); GRAMMENOS, Wassilios, D-67063 Ludwigshafen (DE); GROTE, Thomas, D-67105 Schifferstadt (DE); KIRSTGEN, Reinhard, D-67434 Neustadt (DE); MÜLLER, Bernd, D-67227 Frankenthal (DE); MÜLLER, Ruth, D-67159 Friedelsheim (DE); RÖHL, Franz, D-67105 Schifferstadt (DE); AMMERMANN, Eberhard, D-64646 Heppenheim (DE); HARRIES, Volker, D-67227 Frankenthal (DE); LORENZ, Gisela, D-67434 Hambach (DE); STRATHMANN, Siegfried, D-67117 Limburgerhof (DE); GÖTZ, Norbert, D-67547 Worms (DE)
(86) Internationale Anmeldenummer: EP9604255
(87) Internationale Veröffentlichungsnummer: WO9713747

(56) Entgegenhaltungen:
- WO-A-93/16986
- WO-A-94/14322
- WO-A-95/21153
- US-A- 3 840 596

## Beschreibung

Die vorliegende Erfindung betrifft Oxyaminooximether der Formel I 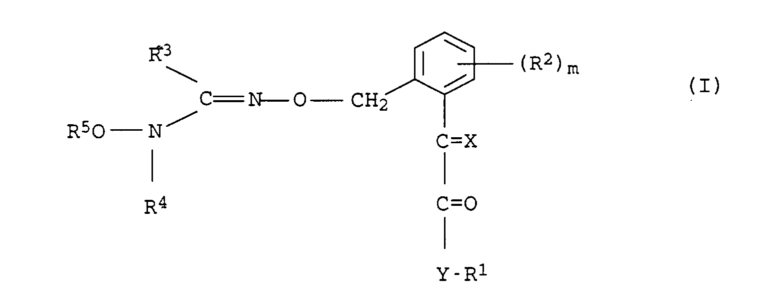 in der die Variablen die folgenden Bedeutungen haben:
- X: NOCH₃, CHOCH₃ oder CHCH₃;
- Y: O oder NZ, wobei Z für Wasserstoff oder C₁-C₄-Alkyl steht;
- R¹: Wasserstoff oder C₁-C₄-Alkyl;
- R²: Cyano, Nitro, Halogen, C₁-C₄-Alkyl, Trifluormethyl oder C₁-C₄-Alkoxy;
- m: 0, 1 oder 2, wobei die Reste R² verschieden sein können, wenn m für 2 steht;
- R³: Wasserstoff, Alkyl, Halogenalkyl oder Cycloalkyl;
- R⁴: Wasserstoff;
C₁-C₆-Alkyl, C₂-C₆-Alkenyl, C₂-C₆-Alkinyl, wobei die drei letztgenannten Reste partiell oder vollständig halogeniert sein und/oder einen bis drei Substituenten tragen können, jeweils ausgewählt aus der Gruppe: Cyano, Nitro, Hydroxy, Mercapto, Amino, Carboxyl, Aminocarbonyl, Aminothiocarbonyl, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Aryl-C₁-C₄-alkoxy, Arylthio, Aryl-C₁-C₄-alkylthio, Heteroaryl, Heteroaryloxy, Heteroaryl-C₁-C₄-alkoxy, Heteroarylthio oder Heteroaryl-C₁-C₄-alkylthio, wobei die cyclischen Gruppen der vierzehn letztgenannten Reste ihrerseits partiell oder vollständig halogeniert sein und/oder einen bis drei Substituenten tragen können, jeweils ausgewählt aus der Gruppe: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Heteroaryl, Heteroaryloxy, Heteroarylthio, C₃-C₆-Alkinyloxy, C₁-C₄-Alkylendioxy, welches halogeniert sein kann, oder C(=NOR⁶)-Aₙ-R⁷, wobei A für Sauerstoff, Schwefel oder Stickstoff steht und wobei der Stickstoff Wasserstoff oder C₁-C₆-Alkyl trägt, n gleich 0 oder 1 ist, R⁶ Wasserstoff oder C₁-C₆-Alkyl und R⁷ Wasserstoff oder C₁-C₆-Alkyl bedeutet;
C₃-C₆-Cycloalkyl, C₄-C₆-Cycloalkenyl, Heterocyclyl, wobei die drei letztgenannten Reste partiell oder vollständig halogeniert sein können und/oder einen bis drei Substitutenten tragen können, jeweils ausgewählt aus der Gruppe: Cyano, C₁-C₆-Alkyl, C₁-C₆-Halogenalkyl, C₂-C₆-Alkenyl, C₁-C₆-Alkoxy und C₁-C₆-Alkylthio;
Aryl oder Heteroaryl, wobei diese beiden Reste partiell oder vollständig halogeniert sein und/oder einen bis drei Substituenten tragen können, jeweils ausgewählt aus der Gruppe: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Alkinyloxy, C₁-C₄-Alkylendioxy, welches halogeniert sein kann, Benzyl, Benzyloxy, Aryl, Aryloxy, Arylthio, Heteroaryl, Heteroaryloxy, Heteroarylthio, wobei die cyclischen Gruppen der acht letztgenannten Reste ihrerseits partiell oder vollständig halogeniert sein können und/oder einen bis drei Substituenten tragen können, jeweils ausgewählt aus der Gruppe: Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyl, C₂-C₆-Alkenyloxy, C₃-C₆-Alkinyloxy und C₁-C₄-Alkylendioxy, welches halogeniert sein kann; und C(=NOR⁶)-Aₙ-R⁷, wobei A für Sauerstoff, Schwefel oder Stickstoff steht und der Stickstoff Wasserstoff oder C₁-C₆-Alkyl trägt, n gleich 0 oder 1 ist, R⁶ Wasserstoff oder C₁-C₆-Alkyl und R⁷ Wasserstoff oder C₁-C₆-Alkyl bedeutet;
- R⁵: Wasserstoff, C₁-C₆-Alkylsulfonyl,
C₁-C₆-Alkyl, C₃-C₆-Alkenyl, C₃-C₆-Alkinyl, wobei diese Reste partiell oder vollständig halogeniert sein können und/oder einen bis drei Substituenten tragen können, jeweils ausgewählt aus der Gruppe: Cyano, Nitro, Hydroxy, Mercapto, Amino, Carboxyl, Aminocarbonyl, Aminothiocarbonyl, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Arylthio, Heteroaryl, Heteroaryloxy und Heteroarylthio, wobei die cyclischen Gruppen der 10 letztgenannten Reste ihrerseits partiell oder vollständig halogeniert sein können und/oder einen bis drei der folgenden Substituenten tragen können, jeweils ausgewählt aus der Gruppe: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Heteroaryl, Heteroaryloxy, Heteroarylthio und C₃-C₆-Alkinyloxy,
C₃-C₆-Cycloalkyl, welches partiell oder vollständig halogeniert sein und/oder eine bis drei Substituenten tragen kann, jeweils ausgewählt aus der Gruppe: Cyano, C₁-C₆-Alkyl, C₁-C₆-Halogenalkyl, C₂-C₆-Alkenyl, C₁-C₆-Alkoxy und C₁-C₆-Alkylthio;
sowie deren Salze.

Außerdem betrifft die Erfindung Verfahren und Zwischenprodukte zur Herstellung dieser Verbindungen sowie sie enthaltende Mittel zur Bekämpfung tierischer Schädlinge und Schadpilze.

Phenylessigsäure-Derivate mit fungizider und insektizider Wirkung sind aus folgenden Druckschriften bekannt: WO-A 93/16986, WO-A 94/14761 und WO-A 94/14322. Strukturell nächstliegend sind die in WO-A 94/14322 beschriebenen Aminooximether. Die erfindungsgemäßen Verbindungen hingegen stellen Oxyaminooximether dar.

Hinsichtlich ihrer Wirkung vermögen die im Stand der Technik offenbarten Wirkstoffe nicht immer zu befriedigen.

Der vorliegenden Erfindung lagen daher neue Verbindungen dieses Typs mit verbesserter Wirkung gegen Schadpilze und Schädlinge als Aufgabe zugrunde.

Demgemäß wurden die eingangs definierten Oxyaminooximether I gefunden. Außerdem wurden Verfahren und Zwischenprodukte zu ihrer Herstellung sowie sie enthaltende Mittel zur Bekämpfung von tierischen Schädlingen und Schadpilzen und ihre Verwendung hierzu gefunden.

Die Verbindungen I sind auf verschiedenen Wegen nach an sich aus der Literatur bekannten Verfahren erhältlich.

Grundsätzlich ist es bei der Synthese der Verbindungen I unerheblich, ob zunächst die Gruppierung -C(X)-CO-YR¹ oder die Gruppierung -CH₂ON=C(R³)-N(R⁴)-OR⁵ aufgebaut wird.

Der Aufbau der Gruppierung -C(X)-CO-YR¹ ist beispielsweise aus folgenden Druckschriften bekannt: EP-A 422 597, EP-A 463 488, EP-A 370 629, EP-A 460 575, EP-A 472 300, WO-A 90/07493, WO-A 92/13830, WO-A 92/18487, DE-A 44 20 416.
1.1 Man geht beim Aufbau der Gruppierung -CH₂ON=C(R³)-N(R⁴)-OR⁵ im allgemeinen so vor, daß man ein Benzylderivat der Formel II mit einem Hydroxyimin der Formel III umsetzt. 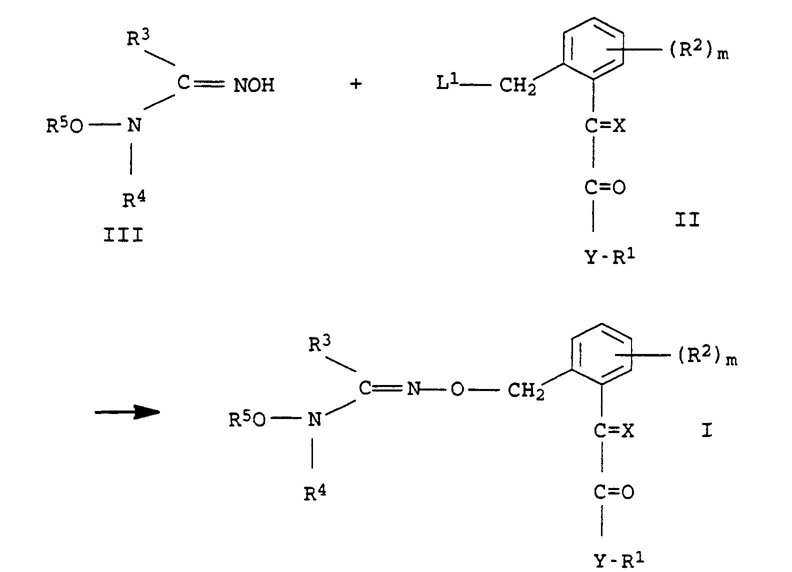
   L¹ in der Formel II steht für eine nukleophil austauschbare Abgangsgruppe, z.B. Halogen oder Sulfonatgruppen, vorzugsweise Chlor, Brom, Iod, Mesylat, Tosylat oder Triflat.
   Die Umsetzung erfolgt in an sich bekannter Weise in einem inerten organischen Lösungsmittel in Gegenwart einer Base, z.B. Natriumhydrid, Kaliumhydroxid, Kaliumcarbonat oder Triethylamin gemäß den in Houben-Weyl, 4. Auflage, Bd. E 14b, S. 370 ff. und Bd. 10/1, S. 1189 ff. beschriebenen Methoden.
1.2 Das benötigte Hydroxyimin III erhält man beispielsweise durch Umsetzung eines Thioamids VII mit Hydroxylamin oder dessen Salz (bevorzugt: Hydroxylammoniumhalogenid). 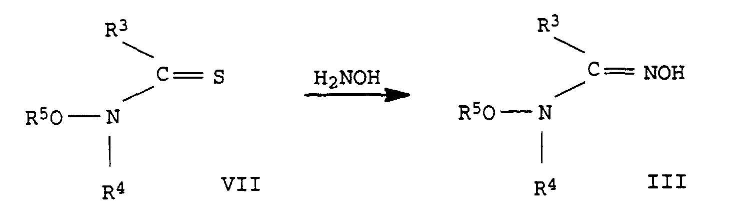
   Die Umsetzung erfolgt in an sich bekannter Weise (vgl. J. Heterocycl. Chem. 17, 819 (1980); Houben-Weyl, 4. Auflage, Band VIII, Seite 694 ff.) in einem inerten organischen Lösungsmittel.
   Einige Thioamide VII sind bekannt (vgl. Acta Cienc. Indica, Chem. 11, 124 (1985); J. Org. Chem. 51, 5198 (1986); J. Org. Chem. 52, 2927 (1987); Tetrahedron Lett. 24, 1851 (1983)), Die Thioamide VIIa sind neu. Sie können in Analogie zu den bekannten Methoden dargestellt werden. 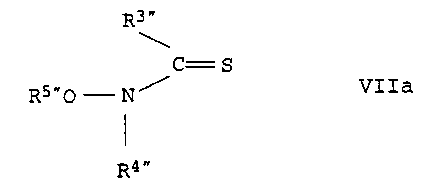
1.3 Eine weitere Möglichkeit zur Herstellung der Hydroxyimine III besteht darin, daß man ein Hydroxylaminderivat der Formel VIII mit einem Hydroximsäurederivat der Formel IX in an sich bekannter Weise in einem inerten organischen Lösungsmittel, ggf. unter Verwendung einer Base, umsetzt (s. Chem.-Ztg. 100, 236 (1976); Chem. Ber. 108, 2764 (1975); J. Org. Chem. 41, 2985 (1976)). 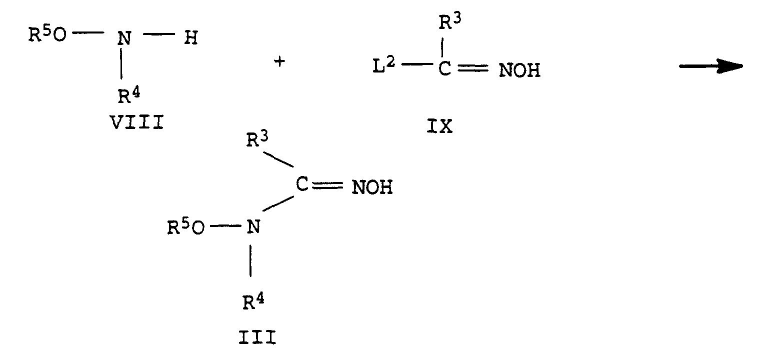
   L² steht für eine verdrängbare Gruppe wie Amino, Halogen, Alkylsulfonyl oder C₁-C₄-Alkoxy, vorzugsweise Amino, Chlor, Brom, Methylsulfonyl, Methoxy oder Ethoxy.
   Die Verbindungen VIII und IX sind bekannt oder können nach an sich bekannten Methoden hergestellt werden (vgl. J. Org. Chem. 47, 1171 (1982); Tetrahedron 48, 6335 (1992); J. Heterocycl. Chem. 12, 1063 (1975); Am. Chem. J. 38, 257 (1907); Tetrahedron 40, 2985 (1984); Helv. Chim. Acta 69, 1137 (1986)).
2. Alternativ können die Verbindungen I auch dadurch erhalten werden, daß das Benzylderivat II zunächst mit dem Hydroxyaminohydroxyiminoderivat IV in ein entsprechendes Benzyloxim der Formel V umgesetzt wird, wobei V anschließend mit einer Verbindung VI zu I umgesetzt wird. 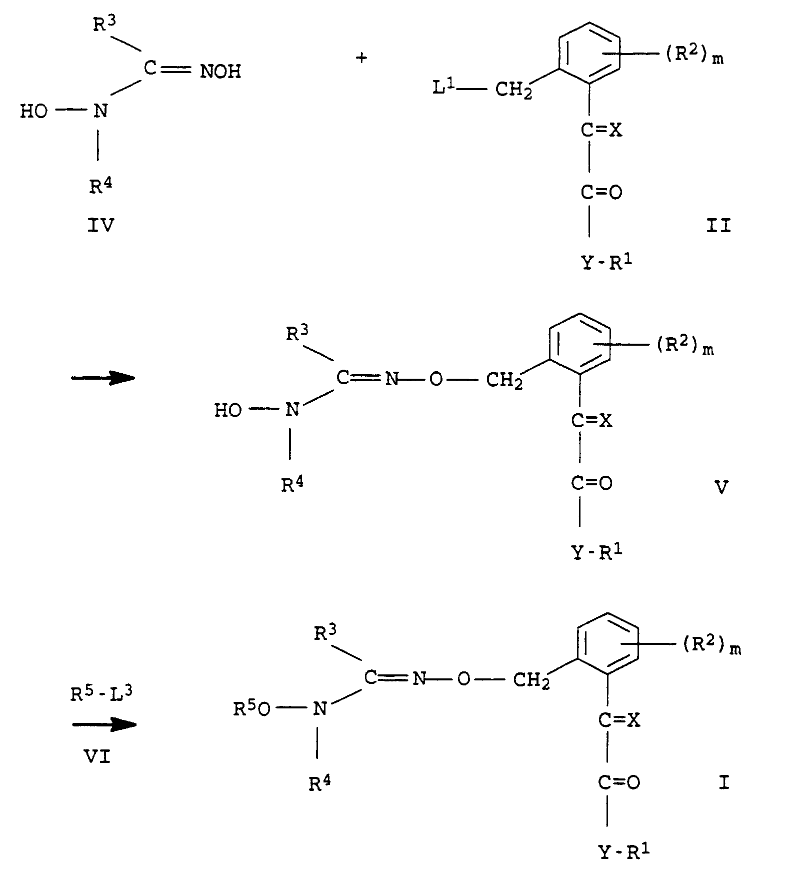
   (L³ = nucleophil austauschbare Abgangsgruppe wie Halogen, z.B. Chlor, Brom, Sulfonat z.B. Mesylat, Trifat, Tesylat)
   Die Umsetzung erfolgt in an sich bekannter Weise in einem inerten organischen Lösungsmittel in Gegenwart einer Base, z.B. Kaliumcarbonat, Kaliumhydroxid, Natriumhydrid, Pyridin oder Triethylamin gemäß den in Houben-Weyl, 4. Auflage, Bd. 10/1, S. 1189 ff.; ibid., Bd. E 14b, S. 307 ff., S. 370 ff. und S. 385 ff.; ibid., Bd. 10/4, S. 55 ff., S. 180 ff. und S. 217 ff.; ibid., Bd. E 5, S. 780 ff. und Farmaco. Ed. Sci. 39, 1060 (1984) beschriebenen Methoden.
   Die Synthese der Verbindungen IV wurde bereits oben beschrieben.
3. Eine weitere Möglichkeit zur Herstellung der Verbindungen I ist die Umsetzung des Benzylderivats II mit N-Hydroxyphthalimid, nachfolgende Hydrazinolyse zum Benzylhydroxylamin IIa und weitere Umsetzung von IIa mit einem Thioamid VII. 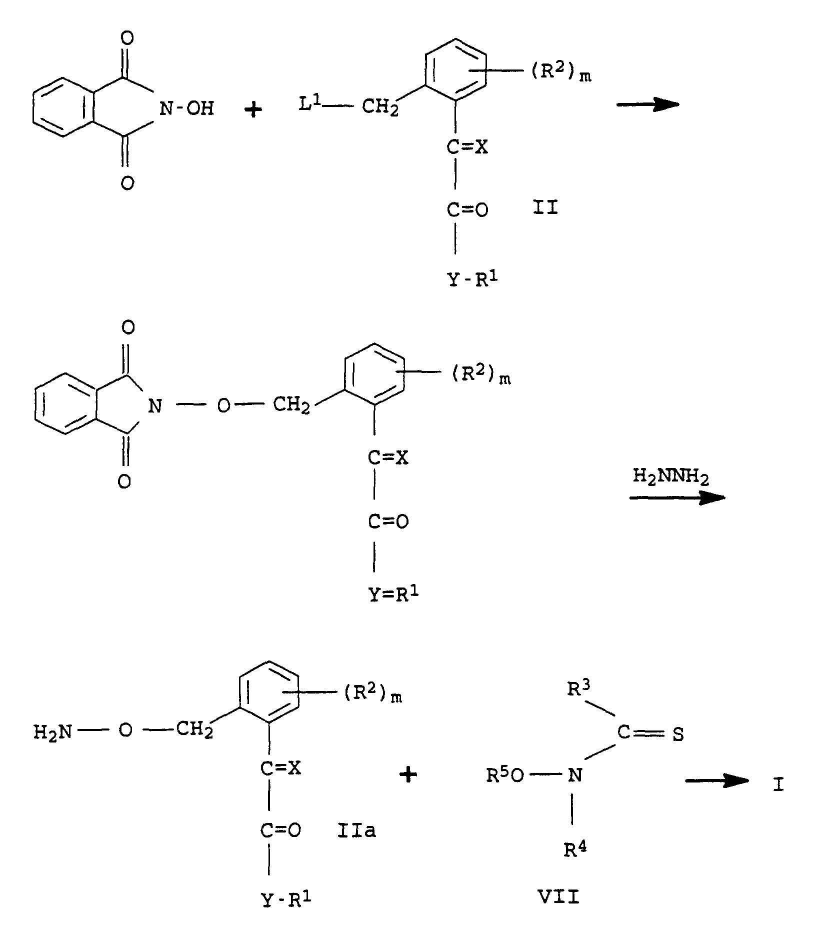
   Die Umsetzung erfolgt in an sich bekannter Weise in einem inerten organischen Lösungsmittel gemäß den in EP-A 463 488 oder DE-A 42 28 867 beschriebenen Methoden oder gemäß der Umwandlung VII → III (s.o.).
4. Entsprechend können die Verbindungen I auch dadurch erhalten werden, daß das Benzylhydroxylamin IIa zunächst mit dem Hydroxythioamid VIIa in das entsprechende Benzyloxyiminoderivat der Formel V umgesetzt wird, wobei V anschließend mit der Verbindung VI wie vorstehend beschrieben zu I umgesetzt wird. 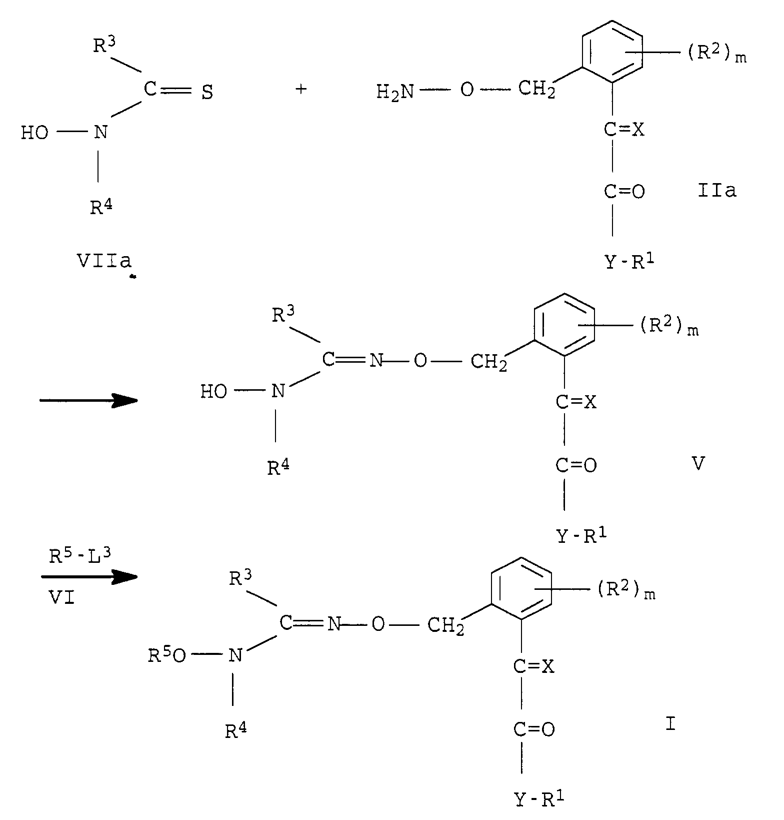
   Dieses Verfahren kann auch zweistufig, wie in Anspruch 4 ausgeführt, durchgeführt werden.
5. Alternativ können die Verbindungen I auch dadurch hergestellt werden, daß man ein Hydroxylaminderivat der Formel VIII mit einem Hydroximsäurederivat der Formel X in an sich bekannter Weise in einem inerten organischen Lösungsmittel umsetzt (vgl. Collect. Czech. Chem. Commun. 48, 596 (1983)). 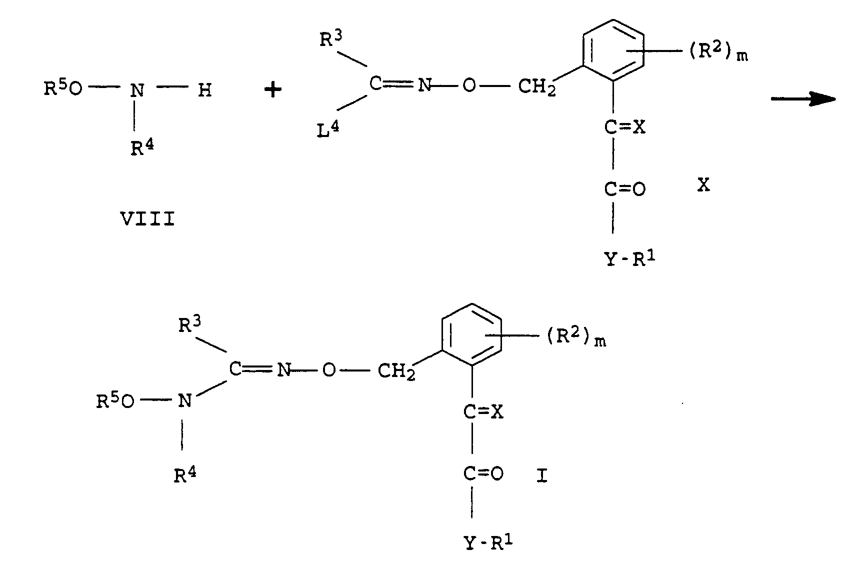
   L⁴ steht für eine verdrängbare Gruppe wie Amino, Halogen, Sulfonyl oder C₁-C₄-Alkoxy, vorzugsweise Amino, Chlor, Brom, Methylsulfonyl, Methoxy oder Ethoxy.
   Die Verbindungen X sind bekannt (vgl. WO-A 94/14761, EP-A 463 488, WO-A 92/18487) oder können nach an sich bekannten Methoden hergestellt werden.
6. Des weiteren erhält man die Verbindungen I dadurch, daß man eine Verbindung III gemäß den in EP-A 493 711 beschriebenen Methoden mit einem Lacton XI zunächst in die entsprechende Benzoesäure XII überführt und XII über die entsprechenden Halogenide XIII in die Cyanocarbonsäuren XIV überführt, welche im Wege der Pinner-Reaktion (Angew. Chem. 94, 1 (1982)) in die α-Ketoester XV überführt und ggf. weiter zu den α-Ketoamiden XVI umgesetzt werden (vgl. EP-A 348 766, DE-A 37 05 389, EP-A 178 826, DE-A 36 23 921, Houben-Weyl, 4. Auflage, Bd. E5, S. 941 ff.). 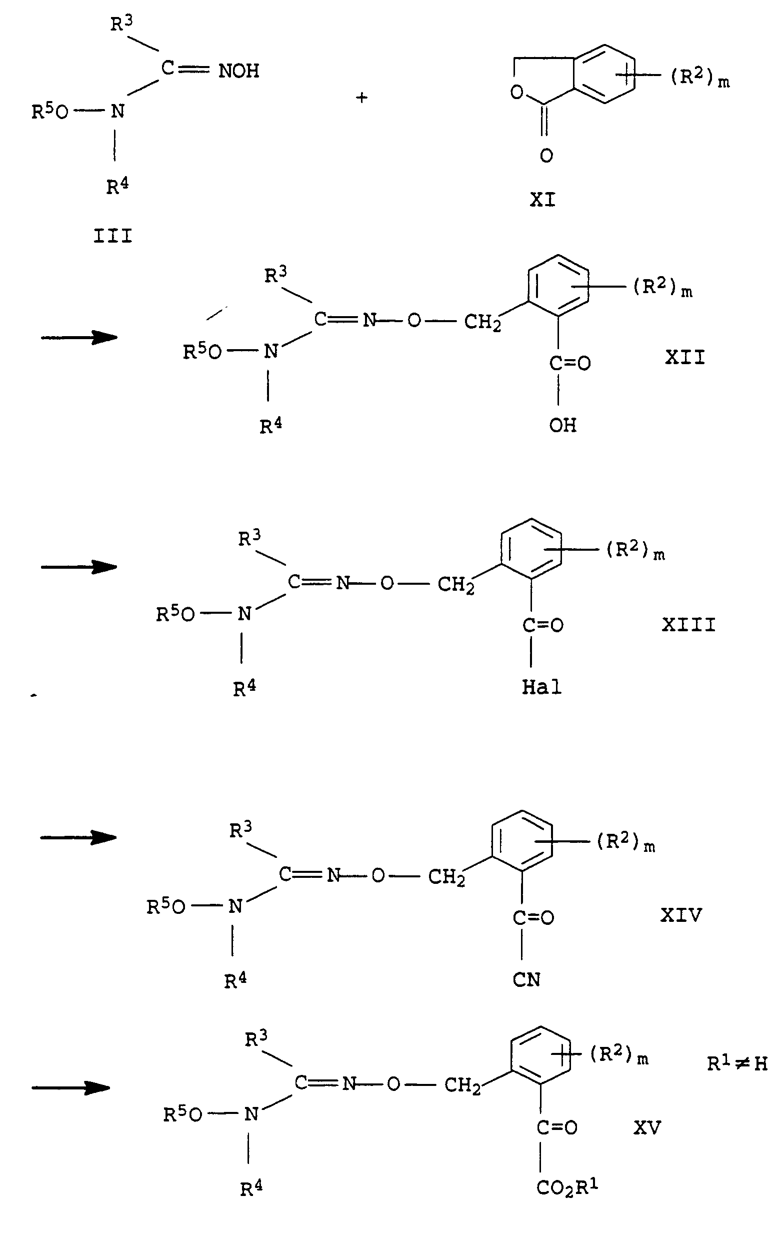 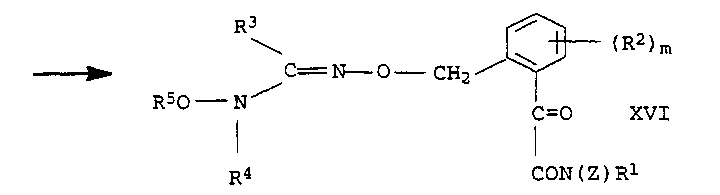 (Z =Wasserstoff oder C₁-C₄-Alkyl)

Die α-Ketoester XV und die α-Ketoamide XVI können gemäß üblichen Verfahren in die Verbindungen I überführt werden (vgl. EP-A 178 826, EP-A 348 766, DE-A 36 23 921, DE-A 37 05 389 sowie die eingangs zitierte Literatur).

Verbindungen I, in denen R¹ Wasserstoff bedeutet, erhält man nach diesem Verfahren durch Verseifung der Ester XV und anschließende Umsetzung zu I.

Die Verbindungen I, in denen Y für NZ steht, können auch aus den Estern (Y=O) durch Umsetzung mit den entsprechenden Aminen HN(Z)R¹ erhalten werden.

Die Verbindungen II sind bekannt aus EP-A 513 580, EP-A 477 631, EP-A 463 488, EP-A 251 082, EP-A 400 417 und/oder EP-A 585 751 oder können nach den dort beschriebenen Methoden hergestellt werden.

Die Verbindungen I können in Form ihrer Salze vorliegen, wobei es auf die Art des Salzes in der Regel nicht ankommt. Die Darstellung der Salze von Verbindungen der Formel I erfolgt auf an sich bekannte Art und Weise.

Die Verbindungen I können bei der Herstellung aufgrund ihrer C=C und C=N Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung der Isomeren nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen, in der behandelten Pflanze oder im zu bekämpfenden Schadpilz oder tierischen Schädling erfolgen.

In Bezug auf die C=X Doppelbindung werden hinsichtlich ihrer Wirksamkeit die E-Isomere der Verbindungen I bevorzugt (Konfiguration bezogen auf die -OCH₃ bzw. die -CH₃ Gruppe im Verhältnis zur -COYR¹ Gruppe).

In Bezug auf die -C(R³)=NOCH₂- Doppelbindung werden hinsichtlich ihrer Wirksamkeit die cis-Isomere der Verbindungen I bevorzugt (Konfiguration bezogen auf den Rest R³ im Verhältnis zur -OCH₂-Gruppe).

Bei der eingangs angegebenen Definitionen der Verbindungen I wurden Sammelbegriffe verwendet, die allgemein repräsentativ für die folgenden Gruppen stehen:
**Halogen:** Fluor, Chlor, Brom und Jod;
**Alkyl:** geradkettige oder verzweigte Alkylgruppen mit 1 bis 4, 1 bis 6 oder 1 bis 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1- Ethyl-2-methylpropyl;
**Alkylamino:** eine Aminogruppe, welche eine geradkettige oder verzweigte Alkylgruppe mit 1 bis 6 Kohlenstoffatomen wie vorstehend genannt trägt;
**Dialkylamino:** eine Aminogruppe, welche zwei voneinander unabhängige, geradkettige oder verzweigte Alkylgruppen mit jeweils 1 bis 6 Kohlenstoffatomen wie vorstehend genannt, trägt;
**Alkylcarbonyl:** geradkettige oder verzweigte Alkylgruppen mit 1 bis 10 Kohlenstoffatomen, welche über eine Carbonylgruppe (-CO-) an das Gerüst gebunden sind;
**Alkylsulfonyl:** geradkettige oder verzweigte Alkylgruppen mit 1 bis 6 oder 1 bis 10 Kohlenstoffatomen, welche über eine Sulfonylgruppe (-SO₂-) an das Gerüst gebunden sind;
**Alkylsulfoxyl:** geradkettige oder verzweigte Alkylgruppen mit 1 bis 6 Kohlenstoffatomen, welche über eine Sulfoxylgruppe (-S(=O)-) an das Gerüst gebunden sind;
**Alkylaminocarbonyl:** Alkylaminogruppen mit 1 bis 6 Kohlenstoffatomen wie vorstehend genannt, welche über eine Carbonylgruppe (-CO-) an das Gerüst gebunden sind;
**Dialkylaminocarbonyl:** Dialkylaminogruppen mit jeweils 1 bis 6 Kohlenstoffatomen pro Alkylrest wie vorstehend genannt, welche über eine Carbonylgruppe (-CO-) an das Gerüst gebunden sind;
**Alkylaminothiocarbonyl:** Alkylaminogruppen mit 1 bis 6 Kohlenstoffatomen wie vorstehend genannt, welche über eine Thiocarbonylgruppe (-CS-) an das Gerüst gebunden sind;
**Dialkylaminothiocarbonyl:** Dialkylaminogruppen mit jeweils 1 bis 6 Kohlenstoffatomen pro Alkylrest wie vorstehend genannt, welche über eine Thiocarbonylgruppe (-CS-) an das Gerüst gebunden sind;
**Halogenalkyl:** geradkettige oder verzweigte Alkylgruppen mit 1 bis 6 Kohlenstoffatomen, wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy:** geradkettige oder verzweigte Alkylgruppen mit 1 bis 4 oder 1 bis 6 Kohlenstoffatomen wie vorstehend genannt, welche über ein Sauerstoffatom (-O-) an das Gerüst gebunden sind, z.B. C₁-C₆-Alkoxy wie Methyloxy, Ethyloxy, Propyloxy, 1-Methylethyloxy, Butyloxy, 1-Methyl-propyloxy, 2-Methylpropyloxy, 1,1-Dimethylethyloxy, Pentyloxy, 1-Methylbutyloxy, 2-Methylbutyloxy, 3-Methylbutyloxy, 2,2-Di-methylpropyloxy, 1-Ethylpropyloxy, Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethyl-butyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy;
**Alkoxycarbonyl:** geradkettige oder verzweigte Alkylgruppen mit 1 bis 6 Kohlenstoffatomen, welche über eine Oxycarbonylgruppe (-OC(=O)-) an das Gerüst gebunden sind;
**Halogenalkoxy:** geradkettige oder verzweigte Alkylgruppen mit 1 bis 6 Kohlenstoffatomen, wobei in diesen Gruppen teilweise oder vollständig die Wasserstoffatome durch Halogenatome wie vorstehend genannt ersetzt sein können, und wobei diese Gruppen über ein Sauerstoffatom an das Gerüst gebunden sind;
**Alkylthio:** geradkettige oder verzweigte Alkylgruppen mit 1 bis 4 oder 1 bis 6 Kohlenstoffatomen wie vorstehend genannt, welche über ein Schwefelatom (-S-) an das Gerüst gebunden sind, z.B. C₁-C₆-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-l-methylpropylthio und 1-Ethyl-2-methylpropylthio;
**Cycloalkyl:** monocyclische Alkylgruppen mit 3 bis 6 Kohlenstoffringgliedern, z.B. Cyclopropyl, Cyclobutyl, Cyclopentyl und Cyclohexyl;
**Alkenyl:** geradkettige oder verzweigte Alkenylgruppen mit 2 bis 6 oder 2 bis 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-l-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1- Ethyl-1-methyl-2-propenyl, l-Ethyl-2-methyl-1-propenyl und 1-Ethyl-2-methyl-2-propenyl;
**Alkenyloxy:** geradkettige oder verzweigte Alkenylgruppen mit 2 bis 6 Kohlenstoffatomen und einer Doppelbindung in einer beliebigen Position, welche über ein Sauerstoffatom (-O-) an das Gerüst gebunden sind;
**Alkenylthio bzw. Alkenylamino:** geradkettige oder verzweigte Alkenylgruppen mit 2 bis 6 Kohlenstoffatomen und einer Doppelbindung in einer beliebigen Position, welche (Alkenylthio) über ein Schwefelatom bzw. (Alkenylamino) ein Stickstoffatom an das Gerüst gebunden sind.
**Alkenylcarbonyl:** geradkettige oder verzweigte Alkenylgruppen mit 2 bis 10 Kohlenstoffatomen und einer Doppelbindung in einer beliebigen Position, welche über eine Carbonylgruppe (-CO-) an das Gerüst gebunden sind;
**Alkinyl:** geradkettige oder verzweigte Alkinylgruppen mit 2 bis 10 Kohlenstoffatomen und einer Dreifachbindung in einer beliebigen Position, z.B. C₂-C₆-Alkinyl wie Ethinyl, 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-l-methyl-2-propinyl;
**Alkinyloxy bzw. Alkinylthio und Alkinylamino:** geradkettige oder verzweigte Alkinylgruppen mit 2 bis 6 Kohlenstoffatomen und einer Dreifachbindung in einer beliebigen Position, welche (Alkinyloxy) über ein Sauerstoffatom bzw. (Alkinylthio) über ein Schwefelatom oder (Alkinylamino) über ein Stickstoffatom an das Gerüst gebunden sind.
**Alkinylcarbonyl:** geradkettige oder verzweigte Alkinylgruppen mit 3 bis 10 Kohlenstoffatomen und einer Dreifachbindung in einer beliebigen Position, welche über eine Carbonylgruppe (-CO-) an das Gerüst gebunden sind;
**Cycloalkenyl bzw. Cycloalkenyloxy, Cycloalkenylthio und Cycloalkenylamino:** monocyclische Alkenylgruppen mit 4 bis 6 Kohlenstoffringgliedern, welche direkt bzw. (Cycloalkenyloxy) über ein Sauerstoffatom oder (Cycloalkenylthio) ein Schwefelatom oder Cycloalkenylamino) über ein Stickstoffatom an das Gerüst gebunden sind, z.B. Cyclobutenyl, Cyclopentenyl und Cyclohexenyl.
**Cycloalkyloxyl bzw. Cycloalkylthio und Cycloalkylamino:** monocyclische Alkylgruppen mit 3 bis 6 Kohlenstoffringgliedern, welche (Cycloalkyloxy) über ein Sauerstoffatom oder (Cycloalkylthio) ein Schwefelatom oder (Cycloalkylamino) über ein Stickstoffatom an das Gerüst gebunden sind, z.B. Cyclopropyl, Cyclobutyl, Cyclopentyl und Cyclohexyl;
**Heterocyclyl bzw. Heterocyclyloxy, Heterocyclylthio und Heterocyclylamino:** drei- bis sechsgliedrige, gesättigte oder partiell ungesättigte mono- oder polycyclische Heterocyclen, die ein bis drei Hereroatome ausgewählt aus einer Gruppe bestehend aus Sauerstoff, Stickstoff und Schwefel enthalten, und welche direkt bzw. (Heterocyclyloxy) über ein Sauerstoffatom oder (Heterocyclylthio) über ein Schwefelatom oder (Heterocyclylamino) über ein Stickstoffatom an das Gerüst gebunden sind, wie 2-Tetrahydrofuranyl, Oxiranyl, 3-Tetrahydrofuranyl, 2-Tetrahydrothienyl, 3-Tetrahydrothienyl, 2-Pyrrolidinyl, 3-Pyrrolidinyl, 3-Isoxazoldinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,3-Dihydro-fur-4-yl, 2,3-Dihydro-fur-5-yl, 2,5-Dihydro-fur-2-yl, 2,5-Dihydro-fur-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydrooxazol-5-yl, 2,3-Dihydroisothiazol-3-yl, 2,3-Dihydroisothiazol-4-yl, 2,3-Dihydroisothiazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydropyrazol-3-yl, 2,3-Dihydropyrazol-4-yl, 2,3-Dihydropyrazol-5-yl, 4,5-Dihydropyrazol-3-yl, 4,5-Dihydropyrazol-4-yl, 4,5-Dihydropyrazol-5-yl, 2,5-Dihydropyrazol-3-yl, 2,5-Dihydropyrazol-4-yl, 2,5-Dihydropyrazol-5-yl, 2,3-Dihydrooxazol-2-yl, 2,3-Dihydrooxazol-4-yl, 2,3-Dihydrooxazol-5-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, 2-Tetrahydropyranyl, 1,3-Dioxolan-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl und 1,3-Dihydrooxazin-2-yl;
**Aryl bzw. Aryloxy, Arylthio, Arylcarbonyl und Arylsulfonyl:** aromatische mono- oder polycyclische Kohlenwasserstoffreste welche direkt bzw. (Aryloxy) über ein Sauerstoffatom (-O-) oder (Arylthio) ein Schwefelatom (-S-), (Arylcarbonyl) über eine Carbonylgruppe (-CO-) oder (Arylsulfonyl) über eine Sulfonylgruppe (-SO₂-) an das Gerüst gebunden sind, z.B. Phenyl, Naphthyl und Phenanthrenyl bzw. Phenyloxy, Naphthyloxy und Phenanthrenyloxy und die entsprechenden Carbonyl- und Sulfonylreste;
**Arylamino:** aromatische mono- oder polycyclische Kohlenwasserstoffreste, wie voranstehend genannt, welche über ein Stickstoffatom an das Gerüst gebunden sind.
**Heteroaryl bzw. Heteroaryloxy, Heteroarylthio, Heteroarylcarbonyl und Hetarylsulfonyl:** aromatische mono- oder polycyclische Reste welche neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten können und welche direkt bzw. (Heteroaryloxy) über ein Sauerstoffatom (-O-) oder (Heteroarylthio) ein Schwefelatom (-S-), (Heteroarylcarbonyl) über eine Carbonylgruppe (-CO-) oder (Heteroarylsulfonyl) über eine Sulfonylgruppe (-SO₂-) an das Gerüst gebunden sind, z.B.
   - 5-gliedriges Heteroaryl. enthaltend ein bis drei Stickstoffatome: 5-Ring Heteroarylgruppen, welche neben Kohlenstoffatomen ein bis drei Stickstoffatome als Ringglieder enthalten können, z.B. 2-Pyrrolyl, 3-Pyrrolyl, 3-Pyrazolyl, 4-Pyrazolyl, 5-Pyrazolyl, 2-Imidazolyl, 4-Imidazolyl, 1,2,4-Triazol-3-yl und 1,3,4-Triazol-2-yl;
   - 5-gliedriges Heteroaryl, enthaltend ein bis vier Stickstoffatome oder ein bis drei Stickstoffatome und ein Schwefel- oder Sauerstoffatom oder ein Sauerstoff oder ein Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder Schwefelatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
   - benzokondensiertes 5-gliedriges Heteroaryl, enthaltend ein bis drei Stickstoffatome oder ein Stickstoffatom und/oder ein Sauerstoff- oder Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder ein Schwefelatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können;
   - über Stickstoff gebundenes 5-gliedriges Heteroaryl, enthaltend ein bis vier Stickstoffatome. oder über Stickstoff gebundenes benzokondensiertes 5-gliedriges Heteroaryl, enthaltend ein bis drei Stickstoffatome: 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, wobei diese Ringe über eines der Stickstoffringglieder an das Gerüst gebunden sind;
   - 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
   - benzokondensiertes 6-gliedrigres Heteroaryl. enthaltend ein bis vier Stickstoffatome: 6-Ring Heteroarylgruppen in welchen zwei benachbarte Kohlenstoffringglieder durch eine Buta-1,3-dien-1,4-diylgruppe verbrückt sein können, z.B. Chinolin, Isochinolin, Chinazolin und Chinoxalin,
   - Pyrido[3,2-d]-thiazol-2-yl;
   bzw. die entsprechenden Oxy-, Thio-, Carbonyl- oder Sulfonylgruppen.
**Hetarylamino:** aromatische mono- oder polycyclische Reste, welche neben Kohlenstoffringgliedern zusätzlich ein bis vier Stickstoffatome oder ein bis drei Stickstoffatome und ein Sauerstoff- oder ein Schwefelatom enthalten können und welche über ein Stickstoffatom an das Gerüst gebunden sind.

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome, wie vorstehend genannt, ersetzt sein können.

Die Angabe "gegebenenfalls substituiert" soll zum Ausdruck bringen, daß in den derart charakterisierten Gruppen die Wasserstoffatome zum Teil oder vollständig durch gleiche oder verschiedene beispielsweise derjenigen Gruppen ersetzt sein können, die unter den vorstehend ausgeführten Sammelbegriffen genannt sind.

Im Hinblick auf ihre biologische Wirkung sind Verbindungen der Formel I bevorzugt, in der die Variablen die folgenden Bedeutungen haben:
- X: NOCH₃, CHOCH₃ oder CHCH₃;
- Y: O oder NZ, wobei Z für Wasserstoff oder C₁-C₄-Alkyl steht;
- R¹: Wasserstoff oder C₁-C₄-Alkyl;
- R²: Cyano, Nitro, Halogen, C₁-C₄-Alkyl, Trifluormethyl oder C₁-C₄-Alkoxy;
- m: 0, 1 oder 2, wobei die Reste R² verschieden sein können, wenn m für 2 steht;
- R³: Wasserstoff, C₁-C₄-Alkyl, C₁-C₄-Halogenalkyl oder Cyclopropyl;
- R⁴: Wasserstoff;
C₁-C₆-Alkyl, C₂-C₆-Alkenyl, C₂-C₆-Alkinyl, wobei die drei letztgenannten Reste partiell oder vollständig halogeniert sein und/oder einen bis drei Substituenten tragen können, jeweils ausgewählt aus der Gruppe: Cyano, Nitro, Hydroxy, Mercapto, Amino, Carboxyl, Aminocarbonyl, Aminothiocarbonyl, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Aryl-C₁-C₄-alkoxy, Arylthio, Aryl-C₁-C₄-alkylthio, Heteroaryl, Heteroaryloxy, Heteroaryl-C₁-C₄-alkoxy, Heteroarylthio oder Heteroaryl-C₁-C₄-alkylthio, wobei die cyclischen Gruppen der vierzehn letztgenannten Reste ihrerseits partiell oder vollständig halogeniert sein und/oder einen bis drei Substituenten tragen können, jeweils ausgewählt aus der Gruppe: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Heteroaryl, Heteroaryloxy, Heteroarylthio, C₃-C₆-Alkinyloxy, C₁-C₄-Alkylendioxy, welches halogeniert sein kann, oder C(=NOR⁶)-Aₙ-R⁷, wobei A für Sauerstoff, Schwefel oder Stickstoff steht und wobei der Stickstoff Wasserstoff oder C₁-C₆-Alkyl trägt, n gleich 0 oder 1 ist, R⁶ Wasserstoff oder C₁-C₆-Alkyl und R⁷ Wasserstoff oder C₁-C₆-Alkyl bedeutet;
C₃-C₆-Cycloalkyl, C₄-C₆-Cycloalkenyl, Heterocyclyl, wobei die drei letztgenannten Reste partiell oder vollständig halogeniert sein können und/oder einen bis drei Substitutenten tragen können, jeweils ausgewählt aus der Gruppe: Cyano, C₁-C₆-Alkyl, C₁-C₆-Halogenalkyl, C₂-C₆-Alkenyl, C₁-C₆-Alkoxy und C₁-C₆-Alkylthio;
Aryl oder Heteroaryl, wobei diese beiden Reste partiell oder vollständig halogeniert sein und/oder einen bis drei Substituenten tragen können, jeweils ausgewählt aus der Gruppe: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Alkinyloxy, C₁-C₄-Alkylendioxy, welches halogeniert sein kann, Benzyl, Benzyloxy, Aryl, Aryloxy, Arylthio, Heteroaryl, Heteroaryloxy, Heteroarylthio, wobei die cyclischen Gruppen der acht letztgenannten Reste ihrerseits partiell oder vollständig halogeniert sein können und/oder einen bis drei Substituenten tragen können, jeweils ausgewählt aus der Gruppe: Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyl, C₂-C₆-Alkenyloxy, C₃-C₆-Alkinyloxy und C₁-C₄-Alkylendioxy, welches halogeniert sein kann; und C(=NOR⁶)-Aₙ-R⁷, wobei A für Sauerstoff, Schwefel oder Stickstoff steht und der Stickstoff Wasserstoff oder C₁-C₆-Alkyl trägt, n gleich 0 oder 1 ist, R⁶ Wasserstoff oder C₁-C₆-Alkyl und R⁷ Wasserstoff oder C₁-C₆-Alkyl bedeutet;
- R⁵: Wasserstoff, C₁-C₆-Alkylsulfonyl,
C₁-C₆-Alkyl, C₃-C₆-Alkenyl, C₃-C₆-Alkinyl, wobei diese Reste partiell oder vollständig halogeniert sein können und/oder einen bis drei Substituenten tragen können, jeweils ausgewählt aus der Gruppe: Cyano, Nitro, Hydroxy, Mercapto, Amino, Carboxyl, Aminocarbonyl, Aminothiocarbonyl, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Arylthio, Heteroaryl, Heteroaryloxy und Heteroarylthio, wobei die cyclischen Gruppen der 10 letztgenannten Reste ihrerseits partiell oder vollständig halogeniert sein können und/oder einen bis drei der folgenden Substituenten tragen können, jeweils ausgewählt aus der Gruppe: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Heteroaryl, Heteroaryloxy, Heteroarylthio und C₃-C₆-Alkinyloxy,
C₃-C₆-Cycloalkyl, welches partiell oder vollständig halogeniert sein und/oder eine bis drei Substituenten tragen kann, jeweils ausgewählt aus der Gruppe: Cyano, C₁-C₆-Alkyl, C₁-C₆-Halogenalkyl, C₂-C₆-Alkenyl, C₁-C₆-Alkoxy und C₁-C₆-Alkylthio;
sowie deren Salze.

Weiterhin sind Verbindungen der Formel I bevorzugt, in denen m für 0 steht.

Gleichermaßen bevorzugt sind Verbindungen der Formel I, in denen R¹ für Methyl steht.

Daneben werden Verbindungen I bevorzugt, in denen R³ für Wasserstoff, Cyclopropyl, Methyl, Ethyl, 1-Methylethyl oder Trifluormethyl steht.

Besonders bevorzugt sind Verbindungen I, in denen R³ für Methyl steht.

Des weiteren werden Verbindungen I bevorzugt, in denen R³ für Trifluormethyl steht.

Des weiteren werden Verbindungen I bevorzugt, in denen R⁴ für gegebenenfalls substituiertes C₁-C₆-Alkyl, C₂-C₆-Alkenyl oder C₂-C₆-Alkinyl steht.

Außerdem werden Verbindungen I bevorzugt, in denen R⁴ für gegebenenfalls substituiertes Heterocyclyl oder gegebenenfalls substituiertes C₄-C₆-Cycloalkenyl steht.

Außerdem werden Verbindungen I bevorzugt, in denen R⁴ für gegebenenfalls substituiertes C₃-C₆-Cycloalkyl steht. Des weiteren werden Verbindungen I bevorzugt, in denen R⁴ für gegebenenfalls substituiertes Aryl oder Hetaryl steht.

Des weiteren werden Verbindungen I bevorzugt, in denen R⁴ für ggf. subst. Pyridyl, Pyrimidyl, Pyrazinyl, Pyridazinyl oder Triazinyl steht.

Des weiteren werden Verbindungen I bevorzugt, in denen R⁴ für ggf. subst. Furyl, Thienyl oder Pyrrolyl steht.

Des weiteren werden Verbindungen I bevorzugt, in denen R⁴ für ggf. subst. Oxazolyl, Thiazolyl, Isoxazolyl, Isothiazolyl, Pyrazolyl oder Imidazolyl steht.

Des weiteren werden Verbindungen I bevorzugt, in denen R⁴ für ggf. subst. Oxdiazolyl, Thiadiazolyl oder Triazolyl steht.

Außerdem werden Verbindungen I bevorzugt, in denen R⁴ für Phenyl steht, welches unsubstituiert ist oder eine oder zwei der folgenden Gruppen trägt: Nitro, Cyano, Hydroxy, Amino, Aminocarbonyl, Aminothiocarbonyl, Halogen, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylamino, C₁-C₄-Alkylcarbonyl, Di-C₁-C₄-Alkylamino, C₁-C₄-Alkylsulfonyl, C₁-C₄-Alkoxycarbonyl, C₁-C₄-Alkylaminocarbonyl, Di-C₁-C₄-Alkylaminocarbonyl, C₁-C₄-Alkylthio, C₂-C₆-Alkenyl, C₂-C₆-Alkenyloxy, C₃-C₆-Alkinyloxy und C₁-C₄-Alkylendioxy, welches halogeniert sein kann.

Des weiteren werden Verbindungen I bevorzugt, in denen R⁵ für C₁-C₆-Alkyl steht.

Außerdem werden Verbindungen I bevorzugt, in denen R⁵ für Methyl oder Ethyl steht.

Des weiteren werden Verbindungen I bevorzugt, in denen R⁵ für Wasserstoff steht.

Außerdem werden Verbindungen I bevorzugt, in denen R⁵ für C₃-C₆-Alkenyl steht.

Außerdem werden Verbindungen I bevorzugt, in denen R⁵ für C₃-C₆-Alkinyl steht.

Daneben werden Verbindungen der Formel I bevorzugt, in denen X für NOCH₃ steht.

Daneben werden Verbindungen der Formel I bevorzugt, in denen X für CHOCH₃ steht.

Daneben werden Verbindungen der Formel I bevorzugt, in denen X für CHCH₃ steht.

Des weiteren werden Verbindungen der Formel I bevorzugt, in denen Y für O steht.

Außerdem werden Verbindungen der Formel I bevorzugt, in denen Y für NH steht.

Insbesondere sind im Hinblick auf ihre Verwendung Verbindungen I bevorzugt, denen die Formeln I.1, I.2, I.3 und I.4 zugrunde liegen, wobei die mit "E" markierten Doppelbindungen E-Konfiguration aufweisen:

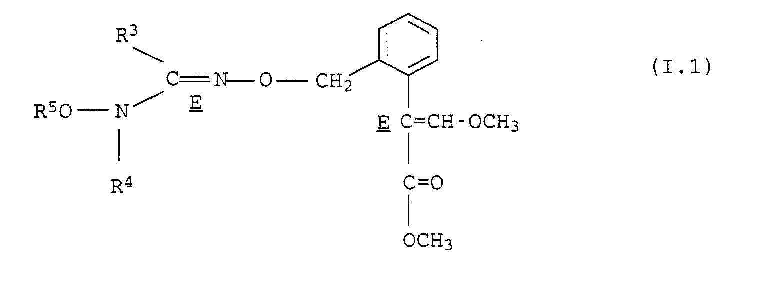

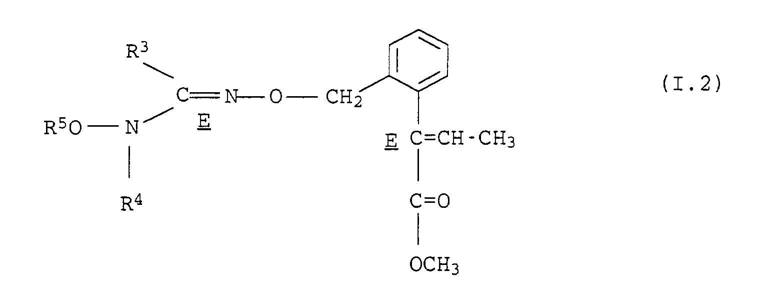

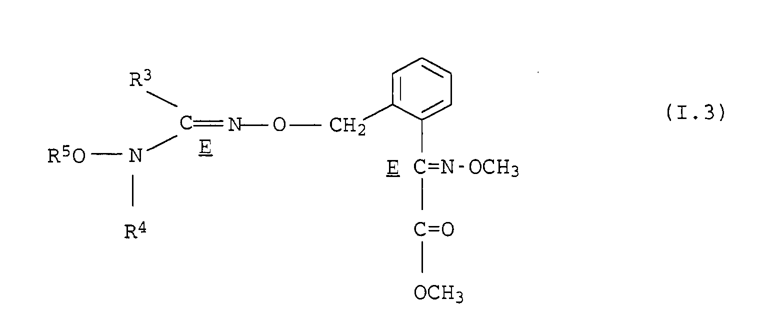

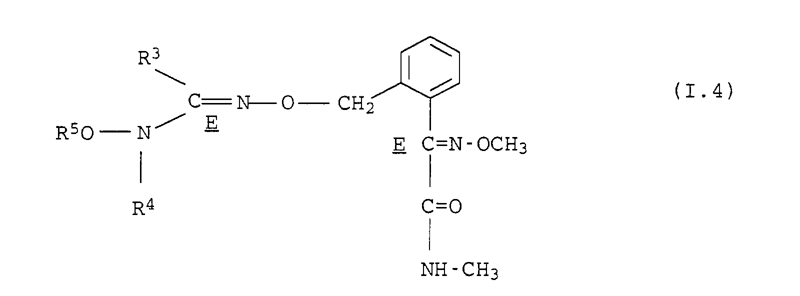

Die Verbindungen IIIa sind neu 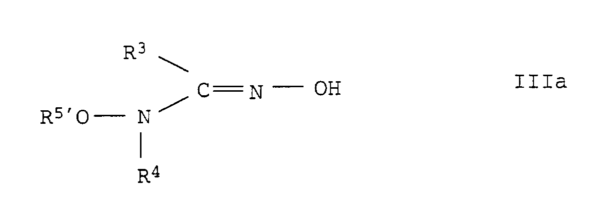 wobei die Variablen R³ und R⁴ die im Anspruch 1 angegebene Bedeutung haben und
- R^{5'}: C₁-C₆-Alkylsulfonyl,
C₁-C₆-Alkyl, C₃-C₆-Alkenyl, C₃-C₆-Alkinyl, wobei diese Reste partiell oder vollständig halogeniert sein können und/oder einen bis drei Substituenten tragen können, jeweils ausgewählt aus der Gruppe: Cyano, Nitro, Hydroxy, Mercapto, Amino, Carboxyl, Aminocarbonyl, Aminothiocarbonyl, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Arylthio, Heteroaryl, Heteroaryloxy und Heteroarylthio, wobei die cyclischen Gruppen der 10 letztgenannten Reste ihrerseits partiell oder vollständig halogeniert sein können und/oder einen bis drei der folgenden Substituenten tragen können, jeweils ausgewählt aus der Gruppe: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Heteroaryl, Heteroaryloxy, Heteroarylthio und C₃-C₆-Alkinyloxy,
C₃-C₆-Cycloalkyl, welches partiell oder vollständig halogeniert sein und/oder eine bis drei Substituenten tragen kann, jeweils ausgewählt aus der Gruppe: Cyano, C₁-C₆-Alkyl, C₁-C₆-Halogenalkyl, C₂-C₆-Alkenyl, C₁-C₆-Alkoxy und C₁-C₆-Alkylthio bedeutet.

Die Synthese erfolgt gemäß den auf S. 3/4 beschriebenen Verfahren, oder können in Analogie zu an sich bekannten Verfahren dargestellt werden.

Die Synthese der Verbindungen VII erfolgt in Analogie zu bekannten Verfahren (vgl. Acta Cienc. Indica. Chem 11, 124 (1985); J. Org. Chem. 51, 5198 (1986); J. Org. Chem. 52, 2927 (1987); Tetrahedron Lett. 24, 1851 (1983).)

Auch neu sind die Verbindungen XII, XIII, XIV, XI, XVI und XVII 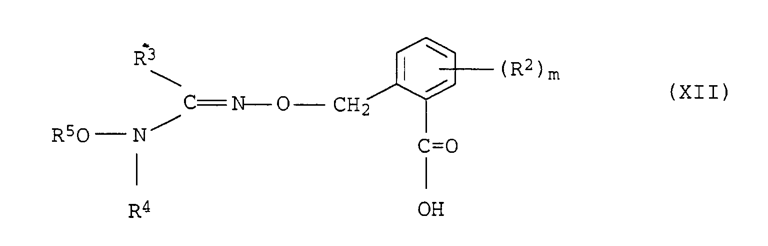 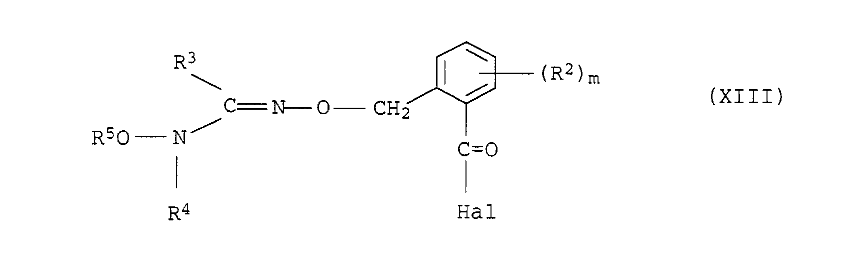 (Hal = Halogen) 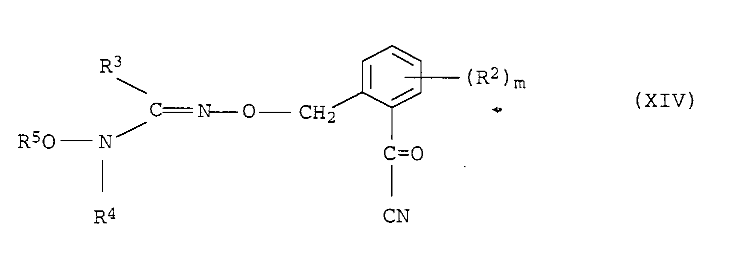 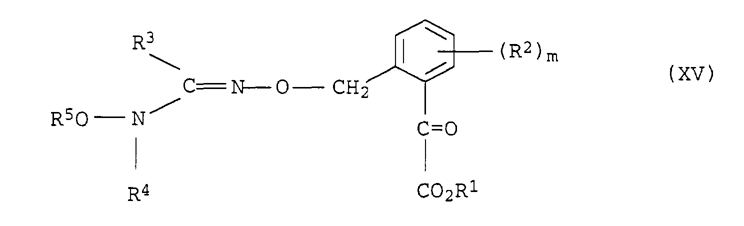 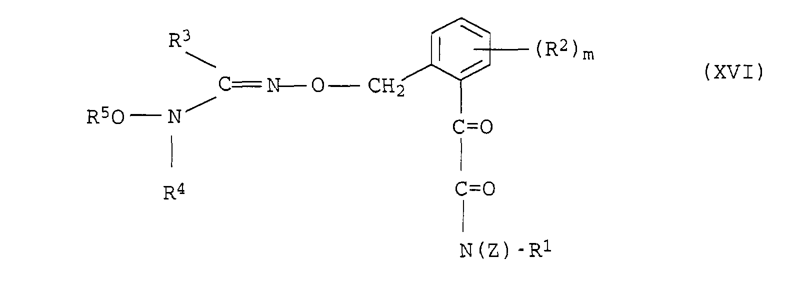 (Z = H oder C₁-C₄-Alkyl)
wobei die Variablen jeweils die gleiche Bedeutung haben wie die entsprechenden Reste von Verbindungen der Formel I. Die Synthese dieser Verbindungen wird auf den Seiten 2 bis 10 dieser Anmeldung beschrieben.

Die Verbindungen I eignen sich als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle, Reis und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Gemüse und Zierpflanzen, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Pseudoperonospora-Arten in Hopfen und Gurken, Alternaria-Arten an Gemüse und Obst.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können mit an sich bekannten Formulierungshilfsmitteln in die üblichen Formulierungen (Mittel) überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der Verbindungen I gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie LigninSulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4-triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,
N-Dichlorfluormethylthio-N',N'-dimethyl-N-phenyl-schwefelsäurediamid, 5-Ethoxy-3-trichlormethyl-1,2,4-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, 2,2,2-trichlor-1-(4-morpholinyl)ethylformamid, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-l-yl)-2-butanon, 1-(4-Chlorphenoxy)-3,3-dimethyl-1-(1H-1,2,4-triazol-1-yl)-2-butanol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alaninmethyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.
Strobilurine wie Methyl-E-methoximino- [α- (o-tolyloxy)-o-tolyl]-acetat, Methyl-E-2-{2-[6-(2-cyanophenoxy)-pyrimidin-4-yloxy]-phenyl}-3-methoxyacrylat, Methyl-E-methoximino-[α-(2-phenoxyphenyl)] -acetamid, Methyl-E-methoximino-[α-(2,5-dimethylphenoxy)-o-tolyl]-acetamid.
Anilinopyrimidine wie N-(4,6-Dimethylpyrimidin-2-yl)-anilin, N-[4-Methyl-6-(1-propinyl)-pyrimidin-2-yl]-anilin, N-(4-Methyl-6-cyclopropyl-pyrimidin-2-yl)-anilin.
Phenylpyrrole wie 4-(2,2-difluor-1,3-benzodioxol-4-yl)-pyrrol-3-carbonitril,
Zimtsäureamide wie 3-(4-Chlorphenyl)-3- (3,4-dimethoxyphenyl)-acrylsäuremorpholid, (2RS,3SR)-1-[3-(2-Chlorphenyl)-2-[4-fluorphenyl]oxiran-2-ylmethyl]-1H-1,2,4-triazol.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nord-mannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Verbindungen I können als solche, in Form ihrer unter Verwendung an sich bekannter Formulierungshilfsmittel gewonnener Formulierungen (Mittel) oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwekken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% mindestens eines Wirkstoffes. Die Wirkstoffe werden dabei in einer Reinheit von 90 % bis 100 %, vorzugsweise 95 % bis 100 % (nach NMR-Spektrum) eingesetzt.

Beispiele für Formulierungen sind:
I. 5 Gew.-Teile einer erfindungsgemäßen Verbindung I werden mit 95 Gew.-Teilen feinteiligem Kaolin innig vermischt. Man erhält auf diese Weise ein Stäubemittel, das 5 Gew.% des Wirkstoffs enthält.
II. 30 Gew.-Teile einer erfindungsgemäßen Verbindung I werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).
III. 10 Gew.-Teile einer erfindungsgemäßen Verbindung I werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).
IV. 20 Gew.-Teile einer erfindungsgemäßen Verbindung I werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).
V. 80 Gew.-Teile einer erfindungsgemäßen Verbindung I werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).
VI. Man vermischt 90 Gew.-Teile einer erfindungsgemäßen Verbindung I mit 10 Gew.-Teilen N-Methyl-α-pyrrolidon und erhält eine Lösung, die zur Anwendung in Form kleinster Tropfen geeignet ist (Wirkstoffgehalt 90 Gew.%).
VII. 20 Gew.-Teile einer erfindungsgemäßen Verbindung I werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.
VIII.20 Gew.-Teile einer erfindungsgemäßen Verbindung I werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiel

Die chemische Verschiebung (in ppm) der ¹H-NMR-Spektren wurde gemessen gegen Tetramethylsilan: br = breit; s = singulett, m = Multiplett.

Darstellung von (E,E)-2-Methoxyimino-2-[2'-[(1"-methyl, 1"-N-methoxy-N-methylamino)iminooxymethyl]phenyl]essigsäure-N-methylamid

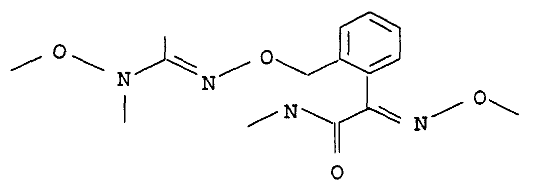

6,3 (61 mmol) N-Methoxy-N-methylacetamid wurden in 100 ml Xylol vorgelegt, mit 24,7 g (61 mmol) 2,4-Bis-(4-methoxyphenyl)-1,3-dithia-2,4-diphosphetan-2,4-disulfid (Lawesson-Reagenz) versetzt und 1 Stunde bei 100 °C gerührt. Das Reaktionsprodukt wurde säulenchromatographisch gereinigt. Man erhielt 5,9 g N-Methoxy-N-methylthioacetamid als orangefarbenes Öl.
¹H-NMR (CDCl₃) : 2,63 (s, 3H); 3,7 (s, 3H); 3,8 (s, 3H).

Zu einer Lösung von 2,7 g (23 mmol) N-Methoxy-N-methylthioacetamid in 50 ml Ethanol gab man 1,9 g (23 mmol) Natriumacetat und 5,4 g (23 mmol) E-2-Methoximino-2-[(2'-aminooxymethyl)phenyl]essigsäuremonomethylamid (vgl. auch die EP-A 585 751) und ließ 16 Stunden bei 70 °C rühren. Das Reaktionsgemisch wurde eingeengt und das Produkt säulenchromatographisch an Kieselgel (Methyl-tert.-butylether/n-Hexan) gereinigt. Man erhielt 1,9 g der Titelverbindung als farbloses Öl.
¹H-NMR (CDCl₃): 1,94 (s, 3H); 2,81 (s, 3H); 2,87 (d, 3H); 3,57 (s, 3H); 3,92 (s, 3H); 4,85 (s, 2H); 6,69 (s, br, 1H); 7,14-7,43 (m, 4H).

### Anwendungsbeispiele

### 1. Beispiel zur Wirkung gegen Schadpilze

Die fungizide Wirkung der Verbindungen der Formel I ließ sich durch folgende Versuche zeigen:

Die Wirkstoffe wurden als 20 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt. Die Auswertung erfolgte visuell.

### Wirksamkeit gegen Weizenmehltau

Blätter von in Töpfen gewachsenen Weizenkeimlingen der Sorte "Frühgold" wurden mit wäßriger Spritzbrühe besprüht und 24 Stunden nach dem Antrocknen des Spritzbelages mit Oidien (Sporen) des Weizenmehltaus (Erysiphe graminis var. tritici) bestäubt. Die Versuchspflanzen wurden anschließend im Gewächshaus bei Temperaturen zw. 20 und 22°C und 75 bis 80 % relativer Luftfeuchtigkeit aufgestellt. Nach 7 Tagen wurde das Ausmaß der Mehltauentwicklung ermittelt.

Nach den oben beschriebenen Methoden wurde die erfindungsgemäße Verbindung, das aus WO-A 93/16986 bekannte Vergleichsmittel A (Tabelle 1, Nr. H-2) und das aus WO-A 94/14761 bekannte Vergleichsmittel B (Tabelle 8, Nr. 1) eingesetzt.

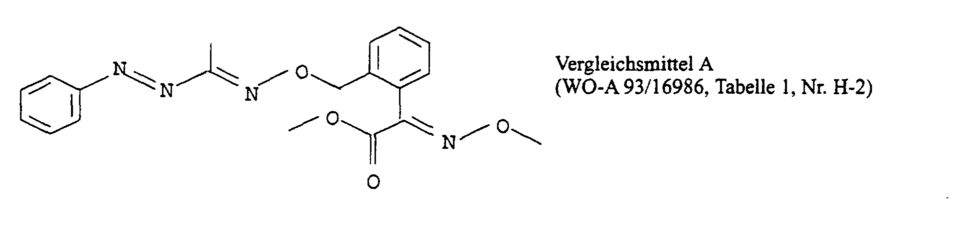

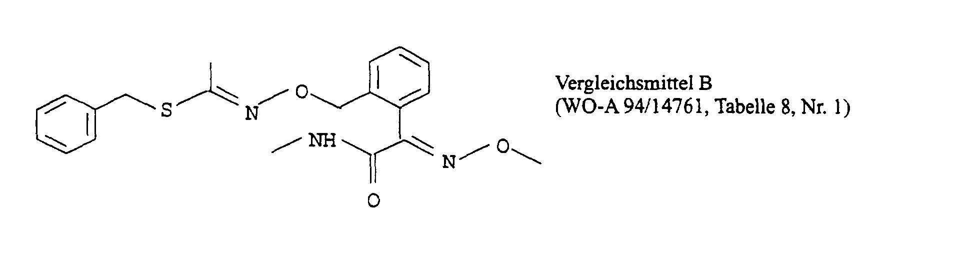

| | %-Befall der Blätter nach Applikation einer wäßrigen Wirkstoffaufbereitung mit 63 ppm Wirkstoff |
|---|---|
| Wirkstoff gemäß Synthesebeispiel | 0 |
| Vergleichsmittel A | 60 |
| Vergleichsmittel B | 40 |
| Unbehandelt | 70 |

Die Ergebnisse der oben genannten Versuche zeigen deutlich die Vorteile der erfindungsgemäßen Verbindung.

### 2. Beispiele zur Wirkung gegen tierische Schädlinge

Die Wirkung der Verbindungen der allgemeinen Formel I gegen tierische Schädlinge ließ sich durch folgende Versuche zeigen

Die Wirkstoffe wurden
a) als 0,1 %-ige Lösung in Aceton oder
b) als 10 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

## Patentansprüche

1. Oxyaminooximether der Formel I 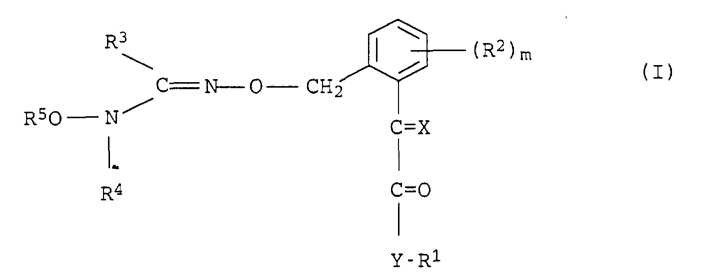 in der die Variablen die folgenden Bedeutungen haben:
X NOCH₃, CHOCH₃ oder CHCH₃;
Y O oder NZ, wobei Z für Wasserstoff oder C₁-C₄-Alkyl steht;
R¹ Wasserstoff oder C₁-C₄-Alkyl;
R² Cyano, Nitro, Halogen, C₁-C₄-Alkyl, Trifluormethyl oder C₁-C₄-Alkoxy;
m 0, 1 oder 2, wobei die Reste R² verschieden sein können, wenn m für 2 steht;
R³ Wasserstoff, Alkyl, Halogenalkyl oder Cycloalkyl;
R⁴ Wasserstoff;
C₁-C₆-Alkyl, C₂-C₆-Alkenyl, C₂-C₆-Alkinyl, wobei die drei letztgenannten Reste partiell oder vollständig halogeniert sein und/oder einen bis drei Substituenten tragen können, jeweils ausgewählt aus der Gruppe: Cyano, Nitro, Hydroxy, Mercapto, Amino, Carboxyl, Aminocarbonyl, Aminothiocarbonyl, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Aryl-C₁-C₄-alkoxy, Arylthio, Aryl-C₁-C₄-alkylthio, Heteroaryl, Heteroaryloxy, Heteroaryl-C₁-C₄-alkoxy, Heteroarylthio oder Heteroaryl-C₁-C₄-alkylthio, wobei die cyclischen Gruppen der vierzehn letztgenannten Reste ihrerseits partiell oder vollständig halogeniert sein und/ oder einen bis drei Substituenten tragen können, jeweils ausgewählt aus der Gruppe: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Heteroaryl, Heteroaryloxy, Heteroarylthio, C₃-C₆-Alkinyloxy, C₁-C₄-Alkylendioxy, welches halogeniert sein kann, oder C(=NOR⁶)-Aₙ-R⁷, wobei A für Sauerstoff, Schwefel oder Stickstoff steht und wobei der Stickstoff Wasserstoff oder C₁-C₆-Alkyl trägt, n gleich 0 oder 1 ist, R⁶ Wasserstoff oder C₁-C₆-Alkyl und R⁷ Wasserstoff oder C₁-C₆-Alkyl bedeutet;
C₃-C₆-Cycloalkyl, C₄-C₆-Cycloalkenyl, Heterocyclyl, wobei die drei letztgenannten Reste partiell oder vollständig halogeniert sein können und/oder einen bis drei Substitutenten tragen können, jeweils ausgewählt aus der Gruppe: Cyano, C₁-C₆-Alkyl, C₁-C₆-Halogenalkyl, C₂-C₆-Alkenyl, C₁-C₆-Alkoxy und C₁-C₆-Alkylthio;
Aryl oder Heteroaryl, wobei diese beiden Reste partiell oder vollständig halogeniert sein und/oder einen bis drei Substituenten tragen können, jeweils ausgewählt aus der Gruppe: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Alkinyloxy, C₁-C₄-Alkylendioxy, welches halogeniert sein kann, Benzyl, Benzyloxy, Aryl, Aryloxy, Arylthio, Heteroaryl, Heteroaryloxy, Heteroarylthio, wobei die cyclischen Gruppen der acht letztgenannten Reste ihrerseits partiell oder vollständig halogeniert sein können und/oder einen bis drei Substituenten tragen können, jeweils ausgewählt aus der Gruppe: Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyl, C₂-C₆-Alkenyloxy, C₃-C₆-Alkinyloxy und C₁-C₄-Alkylendioxy, welches halogeniert sein kann; und C(=NOR⁶)-Aₙ-R⁷, wobei A für Sauerstoff, Schwefel oder Stickstoff steht und der Stickstoff Wasserstoff oder C₁-C₆-Alkyl trägt, n gleich 0 oder 1 ist, R⁶ Wasserstoff oder C₁-C₆-Alkyl und R⁷ Wasserstoff oder C₁-C₆-Alkyl bedeutet;
R⁵ Wasserstoff, C₁-C₆-Alkylsulfonyl,
C₁-C₆-Alkyl, C₃-C₆-Alkenyl, C₃-C₆-Alkinyl, wobei diese Reste partiell oder vollständig halogeniert sein können und/oder einen bis drei Substituenten tragen können, jeweils ausgewählt aus der Gruppe: Cyano, Nitro, Hydroxy, Mercapto, Amino, Carboxyl, Aminocarbonyl, Aminothiocarbonyl, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Arylthio, Heteroaryl, Heteroaryloxy und Heteroarylthio, wobei die cyclischen Gruppen der 10 letztgenannten Reste ihrerseits partiell oder vollständig halogeniert sein können und/oder einen bis drei der folgenden Substituenten tragen können, jeweils ausgewählt aus der Gruppe: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Heteroaryl, Heteroaryloxy, Heteroarylthio und C₃-C₆-Alkinyloxy,
C₃-C₆-Cycloalkyl, welches partiell oder vollständig halogeniert sein und/oder eine bis drei Substituenten tragen kann, jeweils ausgewählt aus der Gruppe: Cyano, C₁-C₆-Alkyl, C₁-C₆-Halogenalkyl, C₂-C₆-Alkenyl, C₁-C₆-Alkoxy und C₁-C₆-Alkylthio;
sowie deren Salze.

2. Verfahren zur Herstellung von Verbindungen der Formel I gemäß Anspruch 1, dadurch gekennzeichnet, daß man ein Benzylderivat der Formel II 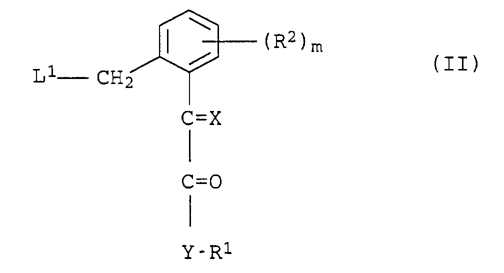 in der L¹ für eine nucleophil austauschbare Abgangsgruppe steht, mit einem Oxim der Formel III 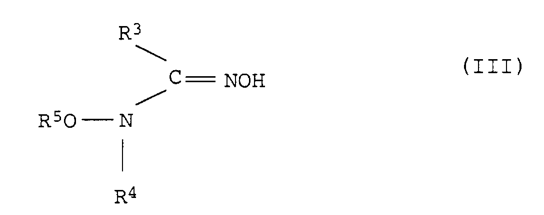 umsetzt.

3. Verfahren zur Herstellung von Verbindungen der Formel I gemäß Anspruch 1, dadurch gekennzeichnet, daß man ein Benzylderivat der Formel II gemäß Anspruch 2 mit einem Hydroxyaminohydroxyimin der Formel IV 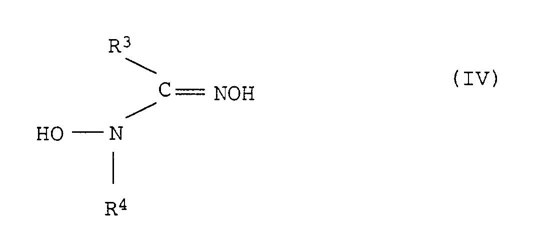 zu einer Verbindung der Formel V 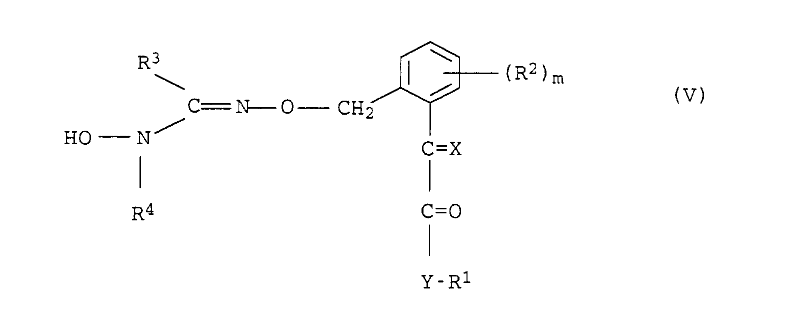 umsetzt und V anschließend mit einer Verbindung der Formel VI
R⁵-L³ (VI)
in der L³ für eine nucleophil austauschbare Abgangsgruppe steht, zu I umsetzt.

4. Verfahren zur Herstellung von Verbindungen der Formel I gemäß Anspruch 1, dadurch gekennzeichnet, daß man ein Benzylhydroxylamin der Formel IIa 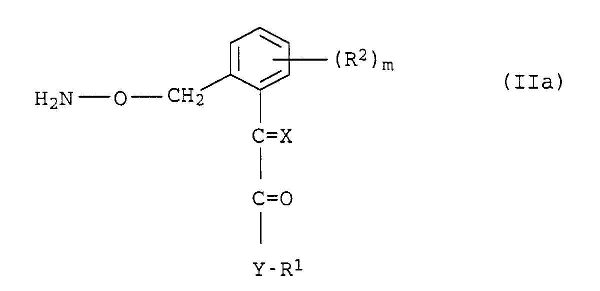 mit einem Thioamid der Formel VII 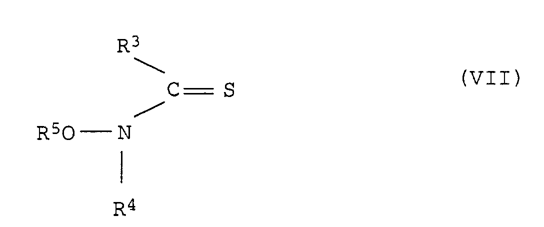 umsetzt.

5. Verfahren zur Herstellung von Verbindungen der Formel I gemäß Anspruch 1, dadurch gekennzeichnet, daß man ein Hydroximsäurederivat der Formel X 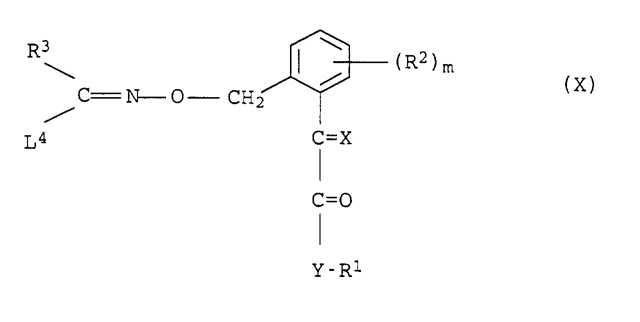 in der L⁴ eine verdrängbare Gruppe bedeutet, mit einem Hydroxylamin der Formel VIII 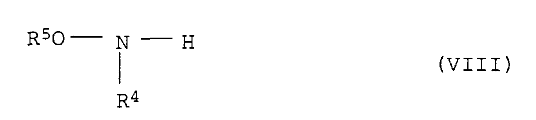 umsetzt.

6. Zur Bekämpfung von tierischen Schädlingen oder von Schadpilzen geeignete Mittel, enthaltend eine Verbindung der Formel I oder eines ihrer Salze gemäß Anspruch 1 und mindestens ein Formulierungshilfsmittel.

7. Mittel nach Anspruch 6 zur Bekämpfung tierischer Schädlinge aus der Klasse der Insekten, Spinntiere oder Nematoden.

8. Verfahren zur Bekämpfung von tierischen Schädlingen oder Schadpilzen, dadurch gekennzeichnet, daß man die Schädlinge oder Schadpilze, deren Lebensraum oder die von ihnen freizuhaltenden Pflanzen, Flächen, Materialien oder Räume mit einer wirksamen Menge einer Verbindung der Formel I oder eines ihrer Salze gemäß Anspruch 1 behandelt.

9. Verfahren zur Herstellung von Mitteln gegen tierische Schädlinge oder gegen Schadpilze, dadurch gekennzeichnet, daß man eine gegen tierische Schädlinge oder gegen Schadpilze wirksame Menge mindestens einer Verbindung der Formel I oder eines seiner Salze, gemäß Anspruch 1, und mindestens einen inerten flüssigen und/oder festen Trägerstoff sowie gewünschtenfalls mindestens einen oberflächenaktiven Stoff mischt.

10. Verwendung der Verbindungen I gemäß Anspruch 1 zur Bekämpfung von tierischen Schädlingen oder von Schadpilzen.

11. Verbindungen der Formel IIIa 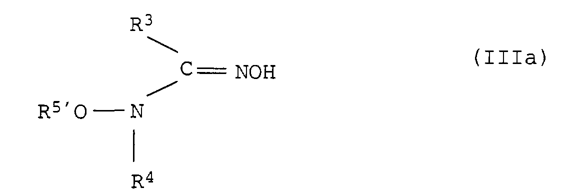 wobei die Variablen R³ und R⁴ die im Anspruch 1 gegebene Bedeutung haben und
R^{5'} die Bedeutung von R⁵ in Anspruch 1 mit Ausnahme von Wasserstoff besitzt.

12. Verbindungen der Formel XII 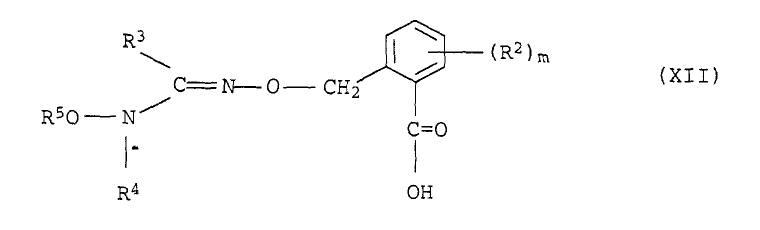 wobei die Variablen die in Anspruch 1 angegebenen Bedeutungen haben.

13. Verbindungen der Formel XIII 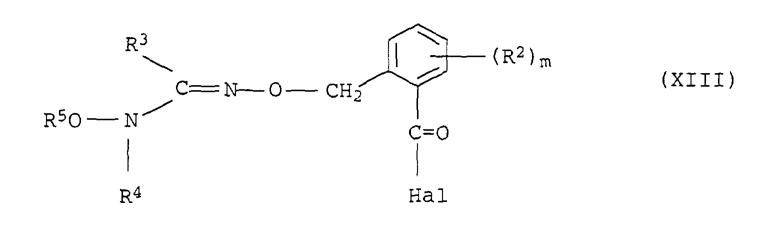 Hal = Halogen, wobei die übrigen Variablen die in Anspruch 1 angegebenen Bedeutungen haben.

14. Verbindungen der Formel XIV 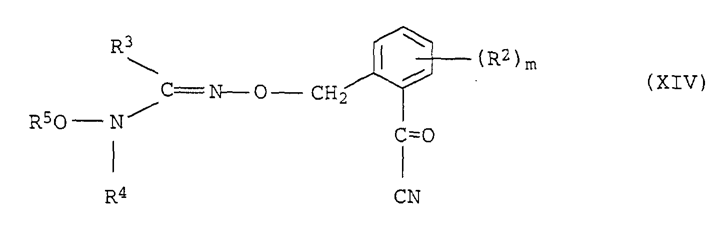 wobei die Variablen die in Anspruch 1 angegebenen Bedeutungen haben.

15. Verbindungen der Formel XV 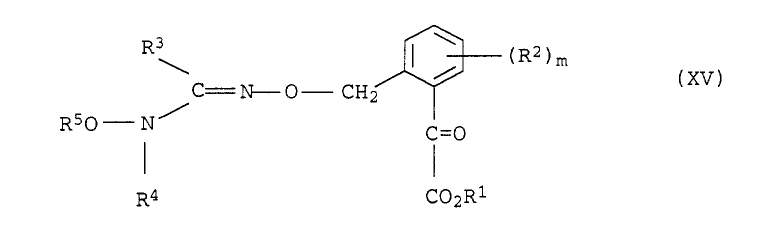 wobei die Variablen die in Anspruch 1 angegebenen Bedeutungen haben.

16. Verbindungen der Formel XVI 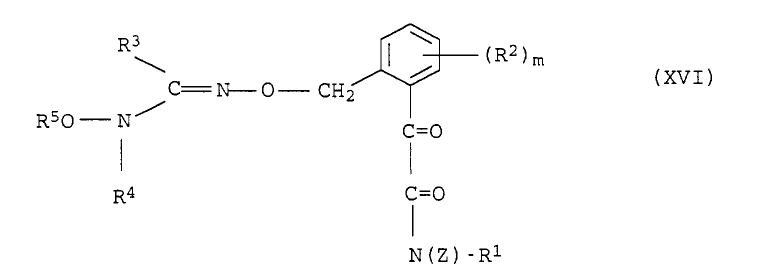 Z = H oder C₁-C₄-Alkyl, wobei die übrigen Variablen die in Anspruch 1 angegebenen Bedeutungen haben.

17. Verwendung der Verbindungen gemäß den Ansprüchen 11 bis 16 als Zwischenprodukte.

## Claims

1. An oxyaminooxime ether of the formula I 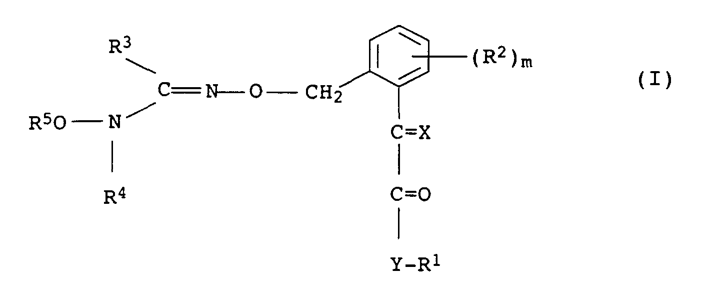 where the variables have the following meanings:
X is NOCH₃, CHOCH₃ or CHCH₃;
Y is O or NZ, Z being hydrogen or C₁-C₄-alkyl;
R¹ is hydrogen or C₁-C₄-alkyl;
R² is cyano, nitro, halogen, C₁-C₄-alkyl, trifluoromethyl or C₁-C₄-alkoxy;
m is 0, 1 or 2, it being possible for the radicals R² to be different if m is 2;
R³ is hydrogen, alkyl, haloalkyl or cycloalkyl;
R⁴ is hydrogen;
C₁-C₆-alkyl, C₂-C₆-alkenyl, C₂-C₆-alkynyl, it being possible for the three last-mentioned radicals to be partially or completely halogenated and/or to carry one to three substituents, in each case selected from the group: cyano, nitro, hydroxyl, mercapto, amino, carboxyl, aminocarbonyl, aminothiocarbonyl, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₁-C₆-alkylsulfonyl, C₁-C₆-alkylsulfoxyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₂-C₆-alkenyloxy, C₃-C₆-cycloalkyl, C₃-C₆-cycloalkyloxy, heterocyclyl, heterocyclyloxy, aryl, aryloxy, aryl-C₁-C₄-alkoxy, arylthio, aryl-C₁-C₄-alkylthio, heteroaryl, heteroaryloxy, heteroaryl-C₁-C₄-alkoxy, heteroarylthio or heteroaryl-C₁-C₄-alkylthio, it being possible for the cyclic groups of the fourteen last-mentioned radicals for their part to be partially or completely halogenated and/or to carry one to three substituents, in each case selected from the group: cyano, nitro, hydroxyl, mercapto, amino, carboxyl, aminocarbonyl, aminothiocarbonyl, C₁-C₆-alkyl, C₁-C₆-haloalkyl, C₁-C₆-alkylcarbonyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, heteroaryl, heteroaryloxy, heteroarylthio, C₃-C₆-alkynyloxy, C₁-C₄-alkylenedioxy, which can be halogenated, or C(=NOR⁶)-Aₙ-R⁷, A being oxygen, sulfur or nitrogen and the nitrogen carrying hydrogen or C₁-C₆-alkyl, n being equal to 0 or 1, R⁶ being hydrogen or C₁-C₆-alkyl and R⁷ being hydrogen or C₁-C₆-alkyl;
C₃-C₆-cycloalkyl, C₄-C₆-cycloalkenyl, heterocyclyl, it being possible for the three last-mentioned radicals to be partially or completely halogenated and/or to carry one to three substituents, in each case selected from the group: cyano, C₁-C₆-alkyl, C₁-C₆-haloalkyl, C₂-C₆-alkenyl, C₁-C₆-alkoxy and C₁-C₆-alkylthio;
aryl or heteroaryl, it being possible for these two radicals to be partially or completely halogenated and/or to carry one to three substituents, in each case selected from the group: cyano, nitro, hydroxyl, mercapto, amino, carboxyl, aminocarbonyl, aminothiocarbonyl, C₁-C₆-alkyl, C₁-C₆-haloalkyl, C₁-C₆-alkylcarbonyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, C₃-C₆-alkynyloxy, C₁-C₄-alkylenedioxy, which can be halogenated, benzyl, benzyloxy, aryl, aryloxy, arylthio, heteroaryl, heteroaryloxy, heteroarylthio, it being possible for the cyclic groups of the eight last-mentioned radicals for their part to be partially or completely halogenated and/or to carry one to three substituents, in each case selected from the group: cyano, nitro, hydroxyl, C₁-C₆-alkyl, C₁-C₆-haloalkyl, C₁-C₆-alkylcarbonyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₂-C₆-alkenyl, C₂-C₆-alkenyloxy, C₃-C₆-alkynyloxy and C₁-C₄-alkylenedioxy, which can be halogenated; and C(=NOR⁶)-Aₙ-R⁷,
A being oxygen, sulfur or nitrogen and the nitrogen carrying hydrogen or C₁-C₆-alkyl, n being equal to 0 or 1, R⁶ being hydrogen or C₁-C₆-alkyl and R⁷ being hydrogen or C₁-C₆-alkyl;
R⁵ is hydrogen, C₁-C₆-alkylsulfonyl,
C₁-C₆-alkyl, C₃-C₆-alkenyl, C₃-C₆-alkynyl, it being possible for these radicals to be partially or completely halogenated and/or to carry one to three substituents, in each case selected from the group: cyano, nitro, hydroxyl, mercapto, amino, carboxyl, aminocarbonyl, aminothiocarbonyl, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₁-C₆-alkylsulfonyl, C₁-C₆-alkylsulfoxyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₂-C₆-alkenyloxy, C₃-C₆-cycloalkyl, C₃-C₆-cycloalkyloxy, heterocyclyl, heterocyclyloxy, aryl, aryloxy, arylthio, heteroaryl, heteroaryloxy and heteroarylthio, it being possible for the cyclic groups of the 10 last-mentioned radicals for their part to be partially or completely halogenated and/or to carry one to three of the following substituents, in each case selected from the group: cyano, nitro, hydroxyl, mercapto, amino, carboxyl, aminocarbonyl, aminothiocarbonyl, C₁-C₆-alkyl, C₁-C₆-haloalkyl, C₁-C₆-alkylcarbonyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, heteroaryl, heteroaryloxy, heteroarylthio and C₃-C₆-alkynyloxy,
C₃-C₆-cycloalkyl, which can be partially or completely halogenated and/or can carry one to three substituents, in each case selected from the group: cyano, C₁-C₆-alkyl, C₁-C₆-haloalkyl, C₂-C₆-alkenyl, C₁-C₆-alkoxy and C₁-C₆-alkylthio;
or its salts.

2. A process for preparing compounds of the formula I as claimed in claim 1, which comprises reacting a benzyl derivative of the formula II 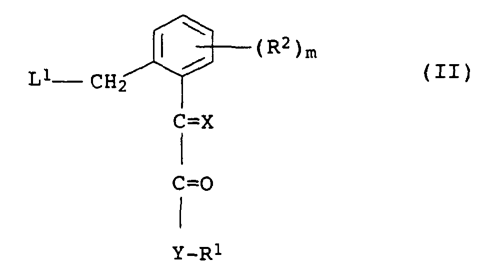 where L¹ is a nucleophilically replaceable leaving group, with an oxime of the formula III

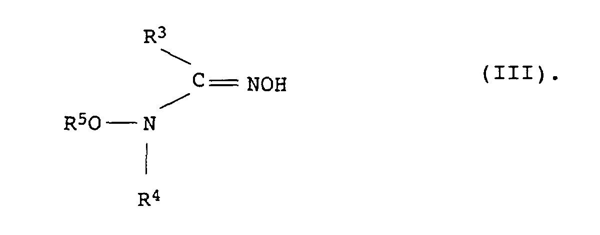

3. A process for preparing compounds of the formula I as claimed in claim 1, which comprises reacting a benzyl derivative of the formula II as claimed in claim 2 with a hydroxyaminohydroxyimine of the formula IV 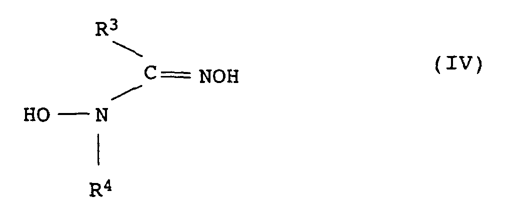 to give a compound of the formula V 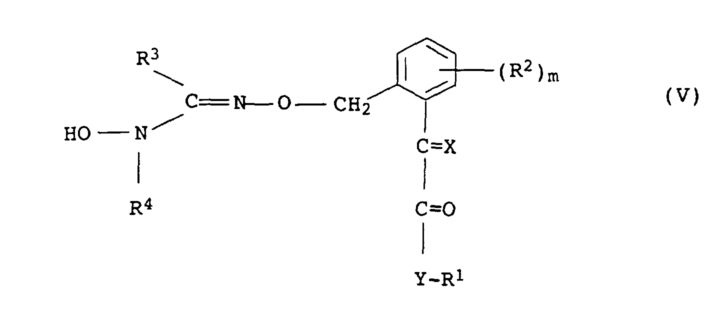 and then reacting V with a compound of the formula VI
R⁵-L³ (VI)
where L³ is a nucleophilically replaceable leaving group, to give I.

4. A process for preparing compounds of the formula I as claimed in claim 1, which comprises reacting a benzylhydroxylamine of the formula IIa 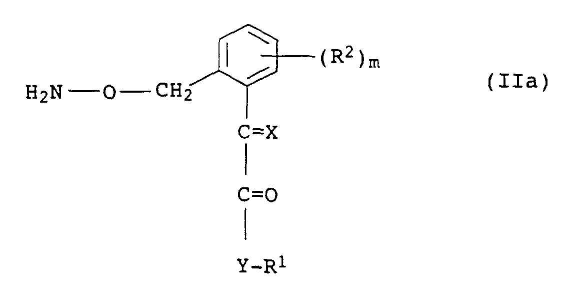 with a thioamide of the formula VII

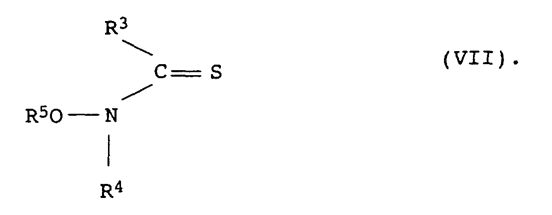

5. A process for preparing compounds of the formula I as claimed in claim 1, which comprises reacting a hydroximic acid derivative of the formula X 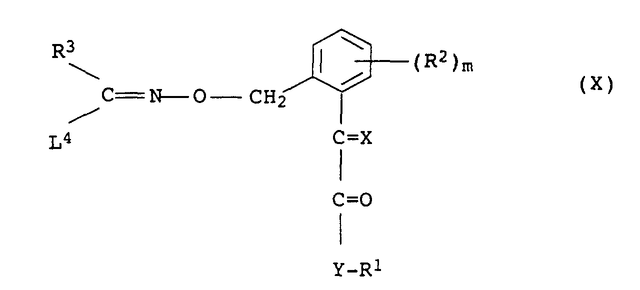 where L⁴ is a displaceable group, with a hydroxylamine of the formula VIII

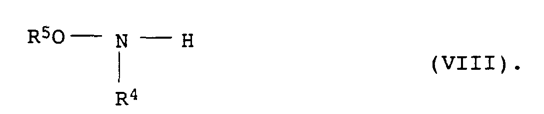

6. A composition suitable for controlling animal pests or harmful fungi, comprising a compound of the formula I or one of its salts as claimed in claim 1 and at least one formulation auxiliary.

7. A composition as claimed in claim 6 for controlling animal pests of the insects, arachnids or nematodes class.

8. A method of controlling animal pests or harmful fungi, which comprises treating the pests or harmful fungi, their habitat or the plants, surfaces, materials or spaces to be kept free from them with an effective amount of a compound of the formula I or one of its salts as claimed in claim 1.

9. A method for preparing compositions against animal pests or against harmful fungi, which comprises mixing an amount of at least one compound of the formula I, or one of its salts, active against animal pests or against harmful fungi, as claimed in claim 1, and at least one inert liquid and/or solid carrier, and, if desired, at least one surface-active substance.

10. The use of the compounds I as claimed in claim 1 for controlling animal pests or harmful fungi.

11. A compound of the formula IIIa 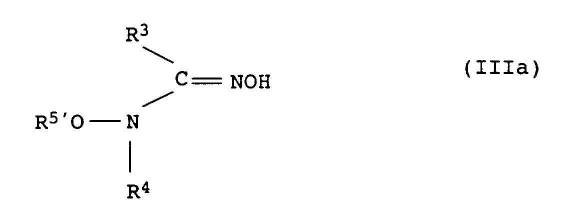 where the variables R³ and R⁴ have the meaning given in claim 1 and
R⁵' has the meaning of R⁵ in claim 1, with the exception of hydrogen.

12. A compound of the formula XII 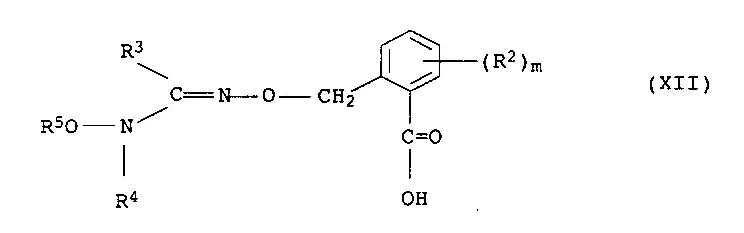 where the variables have the meanings indicated in claim 1.

13. A compound of the formula XIII 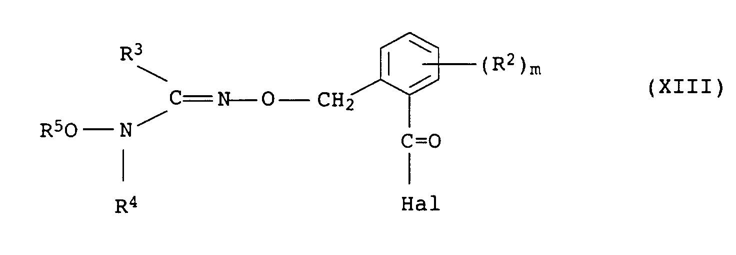 Hal = halogen, where the other variables have the meanings indicated in claim 1.

14. A compound of the formula XIV 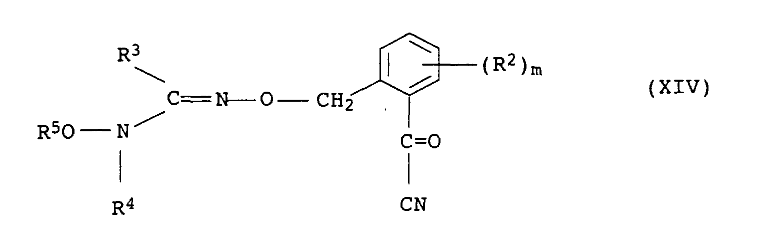 where the variables have the meanings indicated in claim 1.

15. A compound of the formula XV 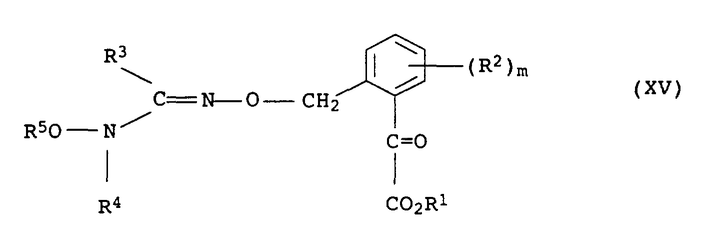 where the variables have the meanings indicated in claim 1.

16. A compound of the formula XVI 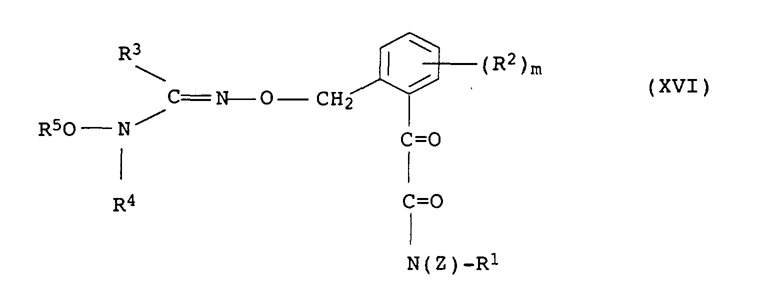 Z = H or C₁-C₄-alkyl, where the other variables have the meanings indicated in claim 1.

17. The use of the compounds as claimed in claims 11 to 16 as intermediates.

## Revendications

1. Ether d'oxyaminooxime de formule I 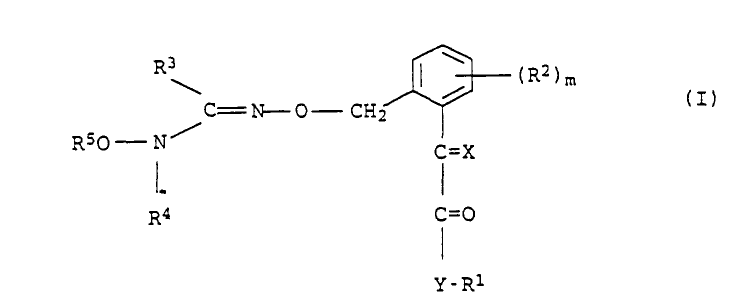 dans laquelle les variables ont les significations suivantes:
X NOCH₃, CHOCH₃ ou CHCH₃;
Y O ou NZ, tandis que Z désigne l'hydrogène ou un alkyle en C₁-C₄;
R¹ hydrogène ou alkyle en C₁-C₄;
R² cyano, nitro, halogéno, alkyle en C₁-C₄, trifluorométhyle ou alcoxy en C₁-C₄;
m 0, 1 ou 2, tandis que les restes R² peuvent être différents lorsque m vaut 2;
R³ hydrogène, alkyle, halogénoalkyle ou cycloalkyle;
R⁴ hydrogène;
alkyle en C₁-C₆, alcényle en C₂-C₆, alcynyle en C₂-C₆, tandis que les trois restes mentionnés en dernier peuvent être partiellement ou totalement halogénés et/ou porter un à trois substituants, dans chaque cas choisis dans le groupe composé de: cyano, nitro, hydroxy, mercapto, amino, carboxyle, aminocarbonyle, aminothiocarbonyle, alkyl(C₁-C₆)aminocarbonyle, dialkyl(C₁-C₆)aminocarbonyle, alkyl(C₁-C₆)-aminothiocarbonyle, dialkyl(C₁-C₆)aminothiocarbonyle, alkylsulfonyle en C₁-C₆, alkylsulfoxyle en C₁-C₆, alcoxy en C₁-C₆, halogénoalcoxy en C₁-C₆, alcoxy(C₁-C₆)carbonyle, alkylthio en C₁-C₆, alkylamino en C₁-C₆, dialkyl-(C₁-C₆)amino, alcényloxy en C₂-C₆, cycloalkyle en C₃-C₆, cycloalkyloxy en C₃-C₆, hétérocyclyle, hétérocyclyloxy, aryle, aryloxy, arylalcoxy(C₁-C₄), arylthio, aryl-alkylthio(C₁-C₄), hétéroaryle, hétéroaryloxy, hétéroaryl-alcoxy-(C₁-C₄), hétéroarylthio ou hétéroaryl-alkylthio(C₁-C₄), tandis que les groupes cycliques des quatorze restes mentionnés en dernier peuvent à leur tour être halogénés partiellement ou totalement et/ou porter un à trois substituants, choisis dans chaque cas dans le groupe composé de: cyano, nitro, hydroxy, mercapto, amino, carboxyle, aminocarbonyle, aminothiocarbonyle, alkyle en C₁-C₆, halogénoalkyle en C₁-C₆, alkyl (C₁-C₆) carbonyle, alkylsulfonyle en C₁-C₆, alkylsulfoxyle en C₁-C₆, cycloalkyle en C₃-C₆, alcoxy en C₁-C₆, halogénoalcoxy en C₁-C₆, alcoxy(C₁-C₆)-carbonyle, alkylthio en C₁-C₆, alkylamino en C₁-C₆, dialkyl(C₁-C₆)amino, alkyl(C₁-C₆) aminocarbonyle, dialkyl(C₁-C₆)aminocarbonyle, alkyl(C₁-C₆)aminothiocarbonyle, dialkyl(C₁-C₆)-aminothiocarbonyle, alcényle en C₂-C₆, alcényloxy en C₂-C₆, benzyle, benzyloxy, aryle, aryloxy, arylthio, hétéroaryle, hétéroaryloxy, hétéroarylthio, alcynyloxy en C₃-C₆, alkylènedioxy en C₁-C₄, qui peut être halogéné, ou C(=NOR⁶)-Aₙ-R⁷, tandis que A désigne l'oxygène, le soufre ou l'azote, et tandis que l'azote porte de l'hydrogène ou un alkyle en C₁-C₆, n vaut 0 ou 1, R⁶ désigne l'hydrogène ou un alkyle en C₁-C₆, et R⁷ désigne l'hydrogène ou un alkyle en C₁-C₆;
cycloalkyle en C₃-C₆, cycloalcényle en C₄-C₆, hétérocyclyle, tandis que les trois restes mentionnés en dernier peuvent être partiellement ou totalement halogénés et/ou porter un à trois substituants, choisis dans chaque cas dans le groupe composé de: cyano, alkyle en C₁-C₆, halogénoalkyle en C₁-C₆, alcényle en C₂-C₆, alcoxy en C₁-C₆ et alkylthio en C₁-C₆;
aryle ou hétéroaryle, tandis que ces deux restes peuvent être partiellement ou totalement halogénés et/ou porter un à trois substituants, choisis dans chaque cas dans le groupe composé de: cyano, nitro, hydroxy, mercapto, amino, carboxyle, aminocarbonyle, aminothiocarbonyle, alkyl en C₁-C₆, halogénoalkyle en C₁-C₆, alkyl(C₁-C₆)carbonyle, alkylsulfonyle en C₁-C₆, alkylsulfoxyle en C₁-C₆, cycloalkyle en C₃-C₆, alcoxy en C₁-C₆, halogénoalcoxy en C₁-C₆, alcoxy(C₁-C₆)carbonyle, alkylthio en C₁-C₆, alkylamino en C₁-C₆, dialkyl(C₁-C₆)amino, alkyl(C₁-C₆)aminocarbonyle, dialkyl (C₁-C₆)-aminocarbonyle, alkyl(C₁-C₆)aminothiocarbonyle, dialkyl(C₁-C₆)aminothiocarbonyle, alcényle en C₂-C₆, alcényloxy en C₂-C₆, alcynyloxy en C₃-C₆, alkylène(C₁-C₄)dioxy, qui peut être halogéné, benzyle, benzyloxy, aryle, aryloxy, arylthio, hétéroaryle, hétéroaryloxy, hétéroarylthio, tandis que les groupes cycliques des huit restes cités en dernier peuvent à leur tour être partiellement ou totalement halogénés et/ou peuvent porter un à trois substituants, à chaque fois choisis dans le groupe composé de: cyano, nitro, hydroxy, alkyle en C₁-C₆, halogénoalkyle en C₁-C₆, alkyl(C₁-C₆)carbonyle, cycloalkyle en C₃-C₆, alcoxy en C₁-C₆, halogénoalcoxy en C₁-C₆, alcoxy(C₁-C₆)carbonyle, alkylthio en C₁-C₆, alkylamino en C₁-C₆, diaikyl(C₁-C₆)amino, alcényle en C₂-C₆, alcényloxy en C₂-C₆, alcynyloxy en C₃-C₆ et alkylène (C₁-C₄)dioxy, qui peut être halogéné; et C(=NOR⁶)-Aₙ-R⁷, tandis que A représente l'oxygène, le soufre ou l'azote et que l'azote porte de l'hydrogène ou un alkyle en C₁-C₆), n vaut 0 ou 1, R⁶ désigne l'hydrogène ou un alkyle en C₁-C₆ et R⁷ représente l'hydrogène ou un alkyle en C₁-C₆;
R⁵ hydrogène, alkylsulfonyle en C₁-C₆,
alkyle en C₁-C₆, alcényle en C₃-C₆, alcynyle en C₃-C₆, tandis que ces restes peuvent être partiellement ou totalement halogénés et/ou peuvent porter un à trois substituants, à chaque fois choisi dans le groupe composé de: cyano, nitro, hydroxy, mercapto, amino, carboxyle, aminocarbonyle, aminothiocarbonyle, alkyl(C₁-C₆)aminocarbonyle, dialkyl(C₁-C₆)-aminocarbonyle, alkyl(C₁-C₆)aminothiocarbonyle, dialkyl(C₁-C₆)aminothiocarbonyle, alkylsulfonyle en C₁-C₆, alkylsulfoxyle en C₁-C₆, alcoxy en C₁-C₆, halogénoalcoxy en C₁-C₆, alcoxy(C₁-C₆)carbonyle, alkylthio en C₁-C₆, alkylamino en C₁-C₆, dialkyl(C₁-C₆)amino, alcényloxy en C₂-C₆, cycloalkyle en C₃-C₆, cycloalkyloxy en C₃-C₆, hétérocyclyle, hétérocyclyloxy, aryle, aryloxy, arylthio, hétéroaryle, hétéroaryloxy et hétéroarylthio, tandis que les groupes cycliques des 10 restes mentionnés en dernier peuvent à leur tour être partiellement ou totalement halogénés et/ou peuvent porter un à trois des substituants suivants, à chaque fois choisis dans le groupe composé de: cyano, nito, hydroxy, mercapto, amino, carboxyle, aminocarbonyle, aminothiocarbonyle, alkyle en C₁-C₆, halogénoalkyle en C₁-C₆, alkyl(C₁-C₆)carbonyle, alkylsulfonyle en C₁-C₆, alkylsulfoxyle en C₁-C₆, cycloalkyle en C₃-C₆, alcoxy en C₁-C₆, halogénoalcoxy en C₁-C₆, alcoxy(C₁-C₆)carbonyle, alkylthio en C₁-C₆, alkylamino en C₁-C₆, dialkyl(C₁-C₆)-amino, alkyl(C₁-C₆)aminocarbonyle, dialkyl-(C₁-C₆)aminocarbonyle, alkyl(C₁-C₆)aminothiocarbonyle, dialkyl(C₁-C₆)aminothiocarbonyle, alcényle en C₂-C₆, alcényloxy en C₂-C₆, benzyle, benzyloxy, aryle, aryloxy, arylthio, hétéroaryle, hétéroaryloxy, hétéroarylthio et alcynyloxy en C₃-C₆,
cycloalkyle en C₃-C₆, qui peut être partiellement ou totalement halogéné et/ou porter un à trois substituants, à chaque fois choisis dans le groupe composé de: cyano, alkyle en C₁-C₆, halogénoalkyle en C₁-C₆, alcényle en C₂-C₆, alcoxy en C₁-C₆ et alkylthio en C₁-C₆;
ainsi que leurs sels.

2. Procédé pour la préparation de composés de formule I selon la revendication 1, caractérisé par le fait qu'on fait réagir un dérivé de benzyle de formule II 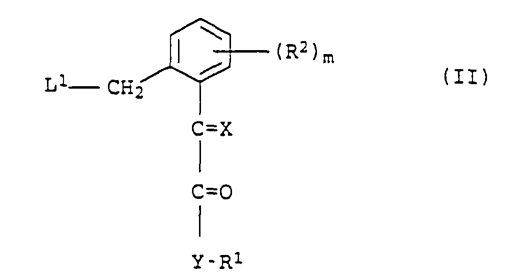 dans laquelle L¹ désigne un groupe labile échangeable par voie nucléophile, avec une oxime de formule III

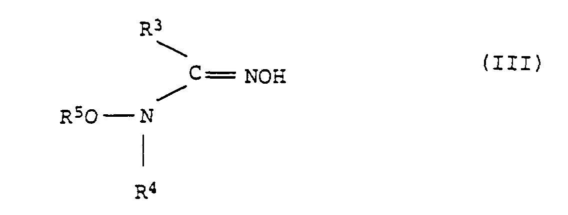

3. Procédé pour la préparation de composés de formule I selon la revendication 1, caractérisé par le fait qu'on fait réagir un dérivé de benzyle de formule II selon la revendication 2 avec une hydroxyaminohydroxyimine de formule IV 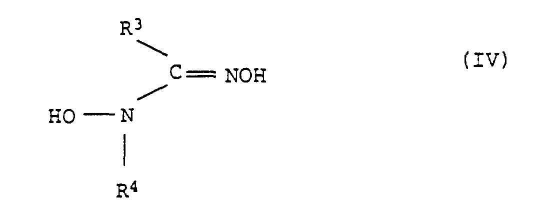 pour former un composé de formule V 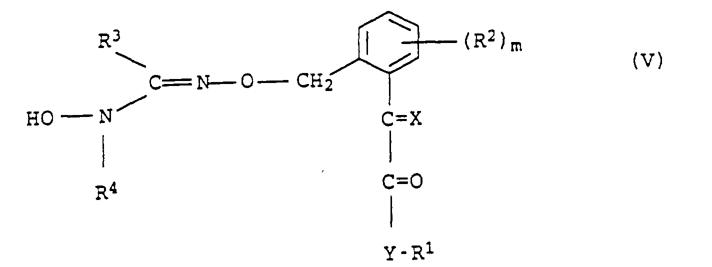 et on transforme ensuite V en I avec un composé de formule VI
R⁵-L³ (VI)
dans laquelle L³ désigne un groupe labile échangeable par voie nucléophile.

4. Procédé pour la préparation de composés de formule I selon la revendication 1, caractérisé par le fait qu'on fait réagir une benzylhydroxylamine de formule IIa 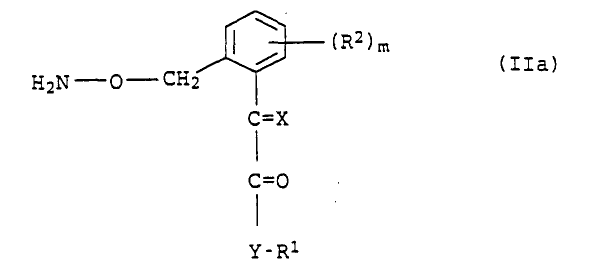 avec un thioamide de formule VII.

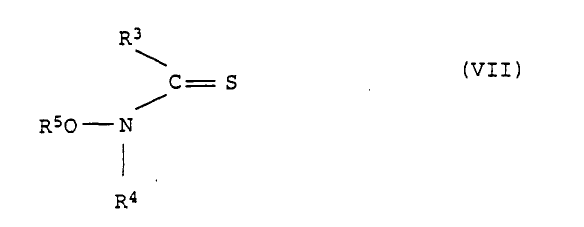

5. Procédé pour la préparation de composés de formule I selon la revendication 1, caractérisé par le fait qu'on fait réagir un dérivé d'acide hydroximique de formule X 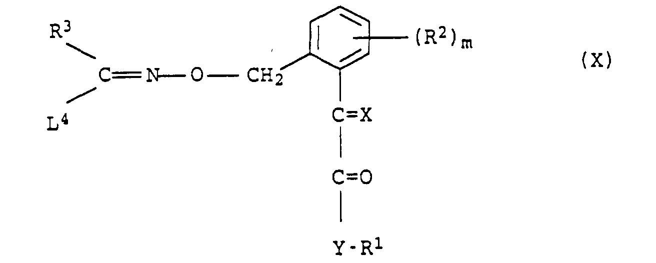 dans laquelle L⁴ désigne un groupe déplaçable, avec une hydroxylamine de formule VIII

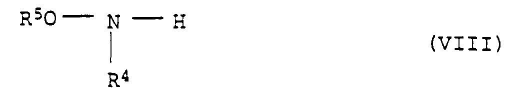

6. Agent approprié pour lutter contre les parasites animaux ou les champignons nuisibles, contenant un composé de formule I ou un de ses sels selon la revendication 1 et au moins un adjuvant de formulation.

7. Agent selon la revendication 6 pour luter contre les parasites animaux de la classe des insectes, des arachnides ou des nématodes.

8. Procédé pour lutter contre des parasites animaux ou des champignons nuisibles, caractérisé par le fait qu'on traite les parasites ou les champignons nuisibles, leur biotope ou les plantes, surfaces, matériaux ou espaces d'où ils doivent être exempts avec une quantité efficace d'un composé de formule I ou d'un sel de celui-ci selon la revendication 1.

9. Procédé pour la préparation d'agents contre les parasites animaux ou contre les champignons nuisibles, caractérisé par le fait qu'on mélange une quantité, efficace contre des parasites animaux ou contre des champignons nuisibles, d'un composé de formule I ou d'un de ses sels, selon la revendication 1, et au moins un support liquide et/ou solide inerte, ainsi qu'éventuellement au moins une substance à activité de surface.

10. Utilisation des composés I selon la revendication 1 pour la lutte contre des parasites animaux ou des champignons nuisibles.

11. Composés de formule IIIa 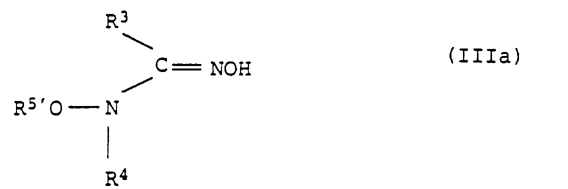 dans lesquels les variables R³ et R⁴ ont la signification indique dans la revendication 1 et
R⁵ possède la signification de R⁵ dans la revendication 1 à l'exception de l'hydrogène.

12. Composés de formule XII 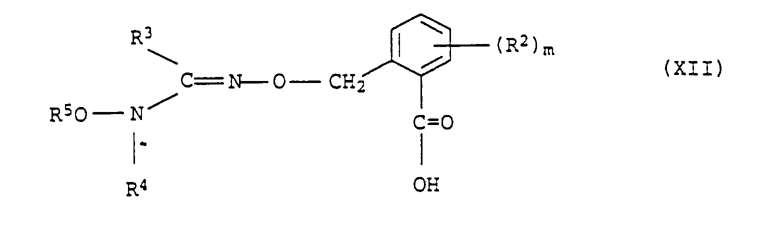 où les variables ont les significations indiquées dans la revendication 1.

13. Composés de formule XIII 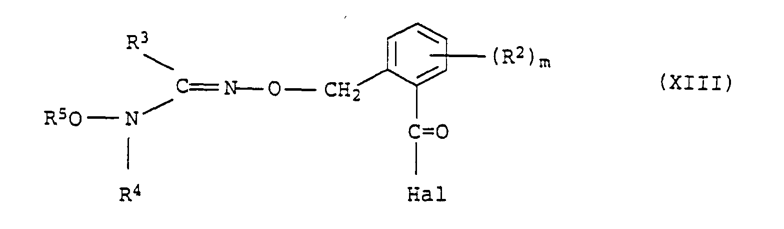 où Hal = halogéno, tandis que les autres variables ont les significations indiquées dans la revendication 1.

14. Composés de formule XIV 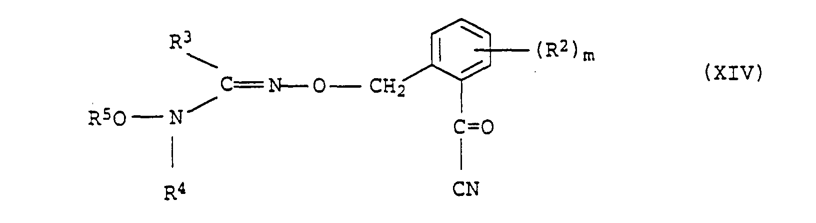 où les variables ont les significations indiquées dans la revendication 1.

15. Composés de formule XV 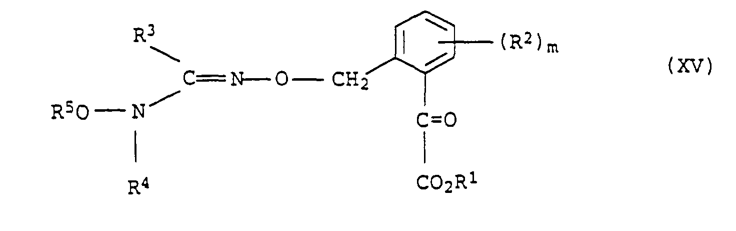 où les variables ont les significations indiquées dans la revendication 1.

16. Composés de formule XVI 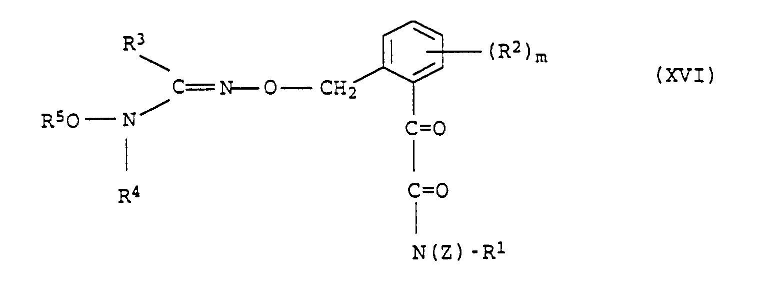 où Z = H ou alkyle en C₁-C₄, tandis que les autres variables ont les significations indiquées dans la revendication 1.

17. Utilisation des composés selon les revendications 11 à 16 en tant que produits intermédiaires.
